# EUROPEAN PATENT SPECIFICATION

(11) **EP 1 743 365 B1**
(45) Date of publication and mention of the grant of the patent: **06.04.2016**
(21) Application number: 05740938.5
(22) Date of filing: 29.04.2005
(51) Int. Cl.: H01L 21/4763, A61M 25/04, A61M 25/06, A61M 25/09, A61B 17/00

(54) **CYSTOTOMY CATHETER CAPTURE DEVICE**
ZYSTOTOMIE-KATHETER-ERFASSUNGSEINRICHTUNG
DISPOSITIF DE SAISIE DE SONDE POUR CYSTOTOMIE

(30) Priority: 03.05.2004 US 837879; 15.01.2005 US 35486
(43) Date of publication of application: 17.01.2007
(73) Proprietor: Swan Valley Medical, INC., Bigfork, MT 59911-2228 (US)
(72) Inventor: HIGH, Kenneth A., Helena, MT 59601 (US)
(74) Representative: Bray, Lilian Janet
(86) International application number: PCT/US2005/015015
(87) International publication number: WO 2005/109487

(56) References cited:
- DE-A1- 3 919 740
- US-A- 3 640 281
- US-A- 3 656 486
- US-A- 3 924 633
- US-A- 5 019 032
- US-A- 5 059 183
- US-A- 5 152 749
- US-A- 5 152 749
- US-A- 5 232 443
- US-A- 5 334 185
- US-A- 5 348 541
- US-A- 5 843 113

## Description

### BACKGROUND OF THE INVENTION

### 1. Field of the Invention.

The present invention relates to the field of medical devices, and more particularly, to a device that facilitates suprapubic catheter placement, even in the morbidly obese, in connection with vaginal surgeries for stress urinary incontinence and pelvic prolapse. The catheter capture device of the present invention can also be used for permanent suprapubic catheterizations in those situations in which patients suffer from incurable incontinence or urinary retention. The catheter capture device of the present invention includes both standard (reusable) and disposable embodiments.

### 2. Description of the Related Art.

A million surgical procedures are performed annually to correct stress urinary incontinence (SUI) in 400,000 American women and 600,000 women abroad. In this context, "stress" refers to sneezing, straining and similar actions that can cause incontinence. A large percentage of these women are unable to void satisfactorily post-operatively and require a catheter to drain the bladder for several days or weeks. Post-operative urinary retention (PUR) occurs in up to forty-one percent (41%) of cases (1, 2). PUR is generally only a temporary event lasting a few days to weeks, but it can be painful, frightening and distressing, and it can complicate postoperative care. In these situations, the suprapubic catheter placed inside-out (I/O)-by passing a sound through the urethra, bladder and abdomen, attaching a catheter to the sound, bringing the catheter into the bladder, and connecting the catheter to drainage-is superior to all other methods. This procedure (called "suprapubic cystotomy") is almost always performed during surgery as opposed to post-operatively because it would require anesthesia and return to the operating room for a second operation.

Suprapubic catheterization may also be necessary with surgeries involving repair of pelvic prolapse, which refers to relaxation of the pelvic floor in a female patient and the descensus or drooping of the bladder, urethra, rectum and/or uterus-whether or not the patient also requires SUI surgery. Approximately seventy percent (70%) of women who undergo SUI surgery also require reconstructive vaginal surgery for repair of pelvic prolapse (3). Thirty percent (30%) of women in the United States and other developed countries experience pelvic prolapse at some point in their lives, and eleven percent (11%) of all women with this condition will require surgery to correct it. Twenty-nine percent (29%) of women who are operated on for prolapse repair will require repeat surgery (4). As these numbers illustrate, the need for a fast and reliable catheter capture method in women undergoing surgery to correct SUI and/or pelvic prolapse is widespread.

When a well functioning suprapubic catheter is in place, accurate post-operative evaluation of bladder recovery and emptying is relatively easy. A typical procedure involves leaving the suprapubic catheter plugged for the first two to three postoperative weeks so that the patient can attempt to void normally and the residual urine can be checked without discomfort. Residual urine is checked by keeping the suprapubic catheter clamped so that the bladder will fill, having the patient void when she feels the need to void, and then removing the plug from the catheter and measuring the urine that drains out of the catheter. When the post-void residual urine is consistently less than 60 ml, the catheter may be removed safely because it is highly unlikely the patient will develop (or redevelop) urinary retention. If urinary retention is still present two to three weeks after surgery, then the catheter can be removed and intermittent self-catheterization (ISC) commenced (5).

ISC is used in one of two situations: (1) when the patient has failed the post-operative trial of voiding and the suprapubic catheter has been removed; or (2) immediately after surgery when a suprapubic catheter was not used. In the former situation, when the patient remains unable to void satisfactorily two to three weeks after surgery, the suprapubic catheter is usually removed, and the patient begins ISC three to four times daily after voiding. With ISC, a new catheter is passed by the patient through the urethra into the bladder each time she voids (to measure the residual urine). When post-void residual urines are low, the patient is free to return to normal voiding without catheters.

The preferred approach is to teach the patient pre-operatively to perform ISC three to four times daily. Many women, however, are either unable to learn or do not want to place a catheter blindly into the urethra and bladder, through a painful, freshly operated area with sutures that are oozing blood and serum (5). The developed consensus among medical practitioners is to place a suprapubic catheter at surgery if the patient has not demonstrated her ability or willingness to perform ISC (1, 6). Passage of a suprapubic catheter from the inside-out (I/O) during surgery is believed to be the best solution because it is safer than passing a catheter from the outside-in (O/I). Furthermore, the I/O technique allows physicians to use larger catheters, which are more reliable in terms of draining the urine. Smaller catheters (*i.e.*, catheters with a smaller diameter-not length) are used with the O/I techniques because O/I can cause perforation of the bowel or peritoneal cavity, and larger tubes (or catheters) would lead to a higher complication rate. The I/O method, despite its advantages, has been awkward and difficult with current devices.

The most commonly employed technique is outside-in (O/I) suprapubic "punch" cystotomy, which entails passage of a small (width) catheter through a small trocar that is "punched" through the abdomen into the bladder. In comparison to I/O techniques, the O/I technique is simple, cheap and easy, but bladder drainage is unreliable because the small catheters often kink or become obstructed when small blood clots enter or form inside the catheter. As a result, the O/I technique is never used for permanent catheterization because of unreliable urine drainage. All O/I devices are more prone to unrecognized bowel or peritoneal perforation with serious secondary complications than the I/O devices. For these reasons, the O/I technique has been condemned by Drs. Ed McGuire and J. Q. Clemens in Campbell's Urology, 8th edition, p. 1160. The applicant believes that an important reason for the current popularity of O/I techniques is because the I/O devices that are currently available are poorly designed, awkward and difficult to use. Moreover, catheter capture is difficult to achieve with these I/O devices.
tip of each device has a short "throw" so that it is difficult to pass the tip of the device through the abdominal wall. When the device is too short to advance through the abdominal wall, catheter capture (*i.e*., securing or affixing the catheter) becomes extremely difficult. Another drawback is that existing catheter capture methods do not work. If the catheter cannot be captured, then the physician will have to insert an indwelling urethral Foley catheter immediately after surgery, or the surgeon will have to make an incision through the abdomen and into the bladder in order to place the suprapubic catheter. Despite the flaws in current technology, there have been no significant developments in catheter placement devices for more than twenty (20) years, although there are a number of patents in this area.

U.S. Patent Nos. 5,152,749 and 5,334,185 (Giesy et al., 1992), 5,232,443 (Leach, 1993) and 5,348,541 (Lyell, 1994) all describe suprapubic catheter placement devices. The Giesy device is limited in that it only describes two means of coupling the catheter to the placement device. These two means are (i) a loop on the catheter and an indentation on the placement device and (ii) a ball and stem on the catheter that fit into a groove and cavity on the placement device. A sheath slides over the device to hold the coupling mechanism in place. US Patent No. 3,656,486 describes a similar arrangement of a hook on the distal end of a catheter inserted through the urethra and a slot on the distal end of a drainage catheter inserted through an incision in the abdominal wall. The hook fits within the slot to pull the drainage catheter in place. A related connection arrangement not involving a hook and a slot is described in US Patent No. 5,019,032. The Leach device is limited in that it has a short "throw" and uses a jaw mechanism to capture the catheter. The jaw mechanism becomes wider after the catheter is enclosed within the jaws, making it more difficult for the catheter to be pulled safely through a small hole in the bladder and potentially resulting in loss of the catheter. Loss of the catheter requires the surgeon to start all over again, subjecting the patient to further unnecessary trauma. The Lyell device is limited in that the only catheter capture means it describes is a hook on the end of a flexible wire. The hook couples with the lateral hole provided in the catheter - not with the hole that extends longitudinally at the tip of the catheter, as in one embodiment of the present invention. German patent DE 3919740, which forms the basis of the preamble of claim 1, describes apparatus used for inserting a drainage catheter in a surgical opening through an abdominal wall. A wire is advanced from a positioning catheter previously inserted through the urethra into the bladder. A balloon is expanded on the end of the positioning catheter once in the bladder. When the surgical opening is formed, the wire is advanced out of the end of the positioning catheter. The balloon is punctured and the wire extended through the incision in the abdominal wall. A drainage catheter is connected to the end of the wire on the outside of the abdominal wall, by connecting together threaded connections on the end of the wire and on the end of an insert which is frictionally held within the end of the drainage catheter. The wire is retracted and the insert expands the size of the opening through the abdominal wall sufficiently to accommodate the drainage catheter. Once the end of the drainage catheter is in the bladder, a sharp and aggressive pull on the wire while holding the drainage catheter removes the insert from the end of the catheter. The positioning catheter, wire and connected insert are pulled through the urethra. US Patent No. 5,843,113 describes another urethra-inserted catheter from which a central element is extended through the abdominal wall. US Patent No. 5,059,183 describes a trocar which confines an obturator therein. Upon penetrating the wall of a hollow organ with the trocar, the obturator is extended from the trocar within the hollow organ, and a distal end of the obturator forms into a hook that prevents back wall penetration of the organ. US Patent No. 3,924,633 describes a wire inside a drainage catheter which, when pulled, causes the end of the drainage catheter to form into a loop that will prevent withdrawal of the catheter. The various embodiments of the present invention are superior to the embodiments described above in terms of efficacy and ease of use.

Because of the problems associated with current suprapubic catheter placement technologies, many patients have been placed on urethral catheterization immediately after surgery instead of suprapubic bladder catheterization during surgery. Urethral catheterization involves placing the catheter directly into the bladder through the urethra. Urethral catheterization is simpler, cheaper and easier than suprapubic catheterization, but it has its disadvantages. Specifically, residual urine is impossible to determine while an indwelling urethral catheter is present to drain the bladder because the catheter fills the urethra and makes it impossible to void. Patients are much more comfortable with suprapubic catheters than with urethral catheters exiting the genitalia, and sexual relations are impossible with a urethral catheter in place. Thus, the preferred alternative is still I/O suprapubic catheter placement, but current methods and available devices are inadequate-particularly in cases involving women, where the distance from the bladder to the abdominal wall is often greater than in men.

Although designed initially to solve problems relating to the use of other catheter placement devices in women, the catheter capture device of the present invention can be used with both women and men. Suprapubic catheterization is often indicated for those men and women who are unable to empty their bladders or who have lost control of their bladders and are required to live in diapers-patients found commonly in nursing homes. These patients include men with high-grade prostate obstruction and men and women with neurologic diseases (such as multiple sclerosis, stroke, Parkinson's disease, Alzheimer's disease and senility) that destroy bladder control and bladder emptying. Most of these patients do not have suprapubic catheterization because it would be a difficult and formidable procedure for them as currently performed.

Accordingly, it is an object of the present invention to provide a fast and reliable device for capturing a suprapubic catheter for placement in the bladder. It is a further object of the present invention to provide a catheter capture device with a "throw" that is sufficiently long to pass through the bladder, abdominal wall and skin easily and rapidly. It is a further object of the present invention to provide a catheter capture device with a modified trocar tip that is suitable for passing over a wire, traversing the abdominal wall, and passing into the bladder. It is a further object of the present invention to provide a catheter capture device that can be used effectively in both women and men whenever suprapubic catheterization is indicated and an abdominal incision is not employed. It is a further object of the present invention to provide a catheter capture device that affords reliable long-term catheter drainage. It is a further object of the present invention to allow placement of permanent suprapubic catheters for chronically ill and elderly men and women instead of condemning them to diapers or long-term urethral catheterization in nursing homes. It is a further object of the present invention to provide a device for placing a suprapubic catheter in the morbidly obese. It is a further object of the present invention to provide a catheter capture device that is disposable.

### BRIEF SUMMARY OF THE INVENTION

According to the present invention there is provided apparatus for creating a suprapubic incision through an abdominal wall into a bladder and for placing a catheter in the incision, comprising in combination:
a hollow urethral sound having a distal end adapted for insertion within a urethra and the bladder and a proximal end adapted to remain outside of the urethra by which to manipulate the distal end of the sound within the bladder, the distal end of the sound having a sound tip;
a wire having proximal and distal ends and positioned within the sound to move between an extended position and a retracted position, the extended position extending the distal end of the wire from the sound tip sufficiently to penetrate from the bladder through the abdominal wall and to expose a portion of the distal end of the wire outside of the abdominal wall, the retracted position retracting the distal end of the wire from the extended position toward the sound tip, the distal end of the wire in the extended position establishing a path for the incision, the movement of the wire from the extended position to the retracted position guiding a distal end of the catheter from outside the abdominal wall through the incision and into the bladder;
a cutting tool adapted to create the incision by movement along the path of the wire through the abdominal wall and into the bladder to the sound tip, the cutting tool is also adapted to be moved out of the incision along the path of the wire after creating the incision;
the catheter having a distal end and a proximal end and a hollow portion extending between the distal end and the proximal end, the distal end of the catheter having a hole which allows liquid to flow into the distal end and through the hollow portion to the proximal end of the catheter, the distal end of the catheter adapted to be placed through the incision and into the bladder while the proximal end of the catheter remains outside of the incision and the abdominal wall, the distal end of the catheter including a catheter tip and an inflatable balloon; and
the cutting tool is adapted to be guided from outside of the abdominal wall along the path of the wire, and the cutting tool is also adapted to be removed from the path of the wire from the outside of the abdominal wall after creating the incision;
a removable connector adapted to selectively connect to the distal end of the wire when the wire is in the extended position, the movement of the wire from the extended position toward the retracted position contacting the connector with the catheter tip and guiding the distal end of the catheter from outside the abdominal wall through the incision and into the bladder, the movement of the distal end of the wire from the retracted position back to the extended position disconnecting the connector from the distal end of the catheter within the bladder the removal of the remaining wire in a distal direction from the incission disconnecting the cathether and the sound and allowing removal of the sound from the urethra;
the removable connector permits the balloon to be inflated within the bladder while the catheter tip remains connected to the connector on the distal end of the wire within the bladder; and
the removable connector permits liquid from the bladder to flow through the hole in the catheter tip into the hollow portion of the catheter to the proximal end while the catheter tip remains connected to the connector on the distal end of the wire within the bladder.

The present invention encompasses a number of embodiments wherein the object may comprise any one of a ball, a nodule, a hook and a crimp in the wire.

A first exemplary embodiment comprises a urethral sound and a sleeve, wherein the urethral sound is inserted through a patient's urethra into the bladder and out the abdominal wall, the sound comprises a removable tip, the removable tip of the sound is removed, the sleeve is attached to the end of the sound from which the removable tip was removed, the catheter to be captured comprises a balloon and a tip, the catheter tip is inserted into the sleeve, the balloon is inflated, and the catheter is pulled into the patient's bladder by pulling the sound out through the patient's urethra. The sleeve optionally comprises a flange that prevents the catheter from falling out of the sleeve when the balloon is inflated.

A second exemplary embodiment comprises a urethral sound and a clamshell device, wherein the urethral sound is inserted through a patient's urethra into the bladder and out the abdominal wall, the clamshell device comprises a top half and a bottom half, the top half of the clamshell device comprises two pegs, the catheter to be captured comprises a tip, the catheter tip comprises two lateral holes, one of the pegs on the top half of the clamshell device passes through the lateral holes in the catheter tip, the bottom half of the clamshell device comprises a notch, the other peg on the top half of the clamshell device fits into the notch on the bottom half of the clamshell device, the clamshell device comprises a threaded end, the sound comprises a distal end, the distal end of the sound is threaded on the inside, the threaded end of the clamshell device fits into the threaded distal end of the sound, and the catheter is pulled into the patient's bladder by pulling the sound out through the patient's urethra.

A third exemplary embodiment comprises a urethral sound, a sleeve and a pin, wherein the urethral sound is inserted through a patient's urethra into the bladder and out the abdominal wall, the urethral sound comprises a threaded extension, the catheter to be captured comprises a tip, the sleeve is placed over the tip of the catheter, the catheter tip comprises lateral holes, the catheter tip is inserted into the threaded extension of the sound, the threaded extension comprises a hole, the pin is inserted through the lateral holes in the catheter tip and through the hole in the threaded extension, the sleeve is threaded on the inside, the sleeve is screwed onto the threaded extension of the sound, and the catheter is pulled into the patient's bladder by pulling the sound out through the patient's urethra.

A fourth embodiment of the present invention comprises a urethral sound, a wire and a ball, wherein the urethral sound is inserted through a patient's urethra into the bladder and out the abdominal wall, the urethral sound comprises a tip, the urethral sound is hollow, a wire is passed through the urethral sound and out the tip, a catheter is placed on the wire, the catheter comprises a proximal end and a distal end, a ball is attached to the wire at the proximal end of the catheter, the catheter comprises a tip, the wire is pulled out through the urethral sound until the ball lodges in the tip of the catheter, and the catheter is pulled into the patient's bladder by pulling the wire and sound out through the patient's urethra.

A fifth embodiment of the present invention comprises a urethral sound, a wire and a hook, wherein the urethral sound is inserted through a patient's urethra into the bladder and out the abdominal wall, the urethral sound comprises a tip, the urethral sound is hollow, a wire is passed through the urethral sound and out the tip, a catheter is placed on the wire, the catheter comprises a proximal end and a distal end, a hook is attached to the wire at the proximal end of the catheter, the catheter comprises a tip, the wire is pulled out through the urethral sound until the hook lodges in the tip of the catheter, and the catheter is pulled into the patient's bladder by pulling the wire and sound out through the patient's urethra.

A sixth embodiment of the present invention comprises a urethral sound, a wire and a nodule, wherein the urethral sound is inserted through a patient's urethra into the bladder and out the abdominal wall, the urethral sound comprises a tip, the urethral sound is hollow, a wire is passed through the urethral sound and out the tip, a catheter is placed on the wire, the catheter comprises a proximal end and a distal end, a nodule is attached to the wire at the proximal end of the catheter, the catheter comprises a tip, the wire is pulled out through the urethral sound until the nodule lodges in the tip of the catheter, and the catheter is pulled into the patient's bladder by pulling the wire and sound out through the patient's urethra.

A seventh embodiment of the present invention comprises a urethral sound and a wire, wherein the urethral sound is inserted through a patient's urethra into the bladder and out the abdominal wall, the urethral sound comprises a tip, the urethral sound is hollow, a wire is passed through the urethral sound and out the tip, the wire comprises a hook, the catheter to be captured comprises a tip, the catheter is placed on the wire such that the hook lodges in the catheter tip, and the catheter is pulled into the patient's bladder by pulling the wire and sound out through the patient's urethra.

An eighth embodiment of the present invention comprises a urethral sound and a wire, wherein the urethral sound is inserted through a patient's urethra into the bladder and out the abdominal wall, the urethral sound comprises a tip, the urethral sound is hollow, a wire is passed through the urethral sound and out the tip, a catheter is placed on the wire, the catheter comprises a proximal end and a distal end, the wire is crimped at the proximal end of the catheter, the catheter comprises a tip, the wire is pulled out through the urethral sound until the crimp in the wire lodges in the tip of the catheter, and the catheter is pulled into the patient's bladder by pulling the wire and sound out through the patient's urethra

The following paragraphs describe a number of different exemplary methods of capturing a catheter corresponding to the embodiments described above. The first method comprises the steps of: inserting a urethral sound through a patient's urethra into the bladder and out the abdominal wall; wherein the urethral sound comprises a removable tip, removing the removable tip; attaching a sleeve to the end of the sound from which the removable tip was removed; wherein the catheter to be captured comprises a tip, inserting the catheter tip into the sleeve; wherein the catheter to be captured comprises a balloon, inflating the balloon; and pulling the catheter into the patient's bladder by pulling the sound out through the patient's urethra. The sleeve optionally comprises a flange that prevents the catheter from falling out of the sleeve when the balloon is inflated.

The second method comprises the steps of: inserting a urethral sound through a patient's urethra into the bladder and out the abdominal wall; wherein the urethral sound comprises a removable tip, removing the removable tip; wherein the catheter to be captured comprises a tip, inserting the catheter tip into the end of the sound from which the removable tip was removed; wherein the catheter to be captured comprises a balloon, inflating the balloon; and pulling the catheter into the patient's bladder by pulling the sound out through the patient's urethra

The third method comprises the steps of: inserting a urethral sound through a patient's urethra into the bladder and out the abdominal wall; wherein the urethral sound comprises a removable tip, removing the removable tip; wherein the catheter to be captured comprises a tip, attaching a clamshell device to the catheter tip; wherein the clamshell device comprises a top half and a bottom half, wherein the top half comprises two pegs, wherein the bottom half comprises a notch, wherein the catheter tip comprises two lateral holes, inserting one of the pegs through the two lateral holes in the catheter tip and inserting the other peg into the notch on the bottom half of the clamshell device; wherein the clamshell device comprises a threaded end, wherein the sound comprises a distal end, wherein the distal end of the sound is threaded on the inside, fitting the threaded end of the clamshell device into the threaded distal end of the sound; and pulling the catheter into the patient's bladder by pulling the sound out through the patient's urethra.

The fourth method comprises the steps of: inserting a urethral sound through a patient's urethra into the bladder and out the abdominal wall; wherein the urethral sound comprises a removable tip, removing the removable tip; wherein the catheter to be captured comprises a tip, placing a sleeve over the catheter tip; wherein the sound comprises a threaded extension, inserting the catheter tip into the threaded extension; wherein the catheter tip comprises two lateral holes, and wherein the threaded extension comprises a hole, inserting a pin through the lateral holes in the catheter tip and the hole in the threaded extension; wherein the sleeve is threaded on the inside, screwing the sleeve onto the threaded extension of the sound; and pulling the catheter into the patient's bladder by pulling the sound out through the patient's urethra.

The fifth method comprises the steps of: inserting a urethral sound through a patient's urethra into the bladder and out the abdominal wall; wherein the urethral sound comprises a tip, and wherein the urethral sound is hollow, passing a wire through the urethral sound and out the tip; placing the catheter to be captured on the wire; wherein the catheter comprises a proximal end and a distal end, attaching a ball to the wire at the proximal end of the catheter; wherein the catheter comprises a tip, pulling the wire out through the urethral sound until the ball lodges in the tip of the catheter; and pulling the catheter into the patient's bladder by pulling the sound out through the patient's urethra.

The sixth method comprises the steps of: inserting a urethral sound through a patient's urethra into the bladder and out the abdominal wall; wherein the urethral sound comprises a tip, and wherein the urethral sound is hollow, passing a wire through the urethral sound and out the tip; placing the catheter to be captured on the wire; wherein the catheter comprises a proximal end and a distal end, attaching a hook to the wire at the proximal end of the catheter; wherein the catheter comprises a tip, pulling the wire out through the urethral sound until the hook lodges in the tip of the catheter; and pulling the catheter into the patient's bladder by pulling the sound out through the patient's urethra.

A seventh method comprises the steps of: inserting a urethral sound through a patient's urethra into the bladder and out the abdominal wall; wherein the urethral sound comprises a tip, and wherein the urethral sound is hollow, passing a wire through the urethral sound and out the tip; placing the catheter to be captured on the wire; wherein the catheter comprises a proximal end and a distal end, attaching a nodule to the wire at the proximal end of the catheter; wherein the catheter comprises a tip, pulling the wire out through the urethral sound until the nodule lodges in the tip of the catheter; and pulling the catheter into the patient's bladder by pulling the sound out through the patient's urethra.

A eighth method comprises the steps of: inserting a urethral sound through a patient's urethra into the bladder and out the abdominal wall; wherein the urethral sound comprises a tip, and wherein the urethral sound is hollow, passing a wire through the urethral sound and out the tip; wherein the catheter comprises a tip, and wherein the wire comprises a hook, placing a catheter on the wire such that the hook lodges in the catheter tip; and pulling the catheter into the patient's bladder by pulling the sound out through the patient's urethra.

A ninth method comprises the steps of: inserting a urethral sound through a patient's urethra into the bladder and out the abdominal wall; wherein the urethral sound comprises a tip, and wherein the urethral sound is hollow, passing a wire through the urethral sound and out the tip; placing the catheter to be captured on the wire; wherein the catheter comprises a proximal end and a distal end, crimping the wire at the proximal end of the catheter; wherein the catheter comprises a tip, pulling the wire out through the urethral sound until the crimp in the wire lodges in the tip of the catheter; and pulling the catheter into the patient's bladder by pulling the sound out through the patient's urethra.

The following paragraphs describe several different exemplary methods of capturing a catheter in an obese patient. The first method comprises the steps of: inserting a urethral sound through a patient's urethra, into the bladder and as close to the abdominal wall as possible; wherein the urethral sound comprises a tip, and wherein the urethral sound is hollow, passing a wire through the urethral sound, out the tip, and through the abdominal wall; placing a knife on the wire; pushing the knife through the abdominal wall until it comes into contact with the tip of the urethral sound; removing the knife; and capturing the catheter using one of the methods described above (ball-on-a-wire, hook-on-a-wire, nodule-on-a-wire, or crimped wire).

The second method in an obese patient comprises the steps of: inserting a urethral sound through a patient's urethra, into the bladder and as close to the abdominal wall as possible; wherein the urethral sound comprises a tip, and wherein the urethral sound is hollow, passing a wire through the urethral sound, out the tip, and through the abdominal Wall; placing a knife on the wire; pushing the knife through the abdominal wall until it comes into contact with the tip of the urethral sound; retracting the urethral sound by at least a distance equal to the length of the tip of the sound; pushing the knife down on the wire again until it comes into contact with the tip of the urethral sound; removing the knife; and capturing the catheter using one of the methods described above (ball-on-a-wire, hook-on-a-wire, nodule-on-a-wire, or crimped wire).

The third method in an obese patient comprises the steps of: inserting a urethral sound through patient's urethra, into the bladder and as close to the abdominal wall as possible; wherein the urethral sound comprises a tip, and wherein the urethral sound is hollow, passing a wire through the urethral sound, out the tip, and through the abdominal wall; placing a trocar on the wire; pushing the trocar through the abdominal wall until it comes into contact with the tip of the urethral sound; removing the trocar; and capturing the catheter using one of the methods described above (ball-on-a-wire, hook-on-a-wire, nodule-on-a-wire, or crimped wire).

The fourth method in an obese patient comprises the steps of: inserting a urethral sound through a patient's urethra, into the bladder and as close to the abdominal wall as possible; wherein the urethral sound comprises a tip, and wherein the urethral sound is hollow, passing a wire through the urethral sound, out the tip, and through the abdominal wall; placing a trocar on the wire; pushing the trocar through the abdominal wall until it comes into contact with the tip of the urethral sound; retracting the urethral sound by at least a distance equal to the length of the tip of the sound; pushing the trocar down on the wire again until it comes into contact with the tip of the urethral sound; removing the trocar; and capturing the catheter using one of the methods described above (ball-on-a-wire, hook-on-a-wire, nodule-on-a-wire, or crimped wire).

The fifth method in an obese patient comprises the steps of: creating an incision through the abdominal wall of an obese patient using a trocar or knife, as described above; after the trocar or knife is removed, placing a screwdriver on the wire; pushing the screwdriver down on the wire until it comes into contact with the tip of the urethral sound; wherein the tip of the urethral sound comprises one or more slots, wherein the screwdriver comprises a tip, wherein the tip of the screwdriver comprises one or more blades, fitting the screwdriver blade(s) into the slot(s) on the tip of the sound; unscrewing the tip of the sound with the screwdriver; removing the screwdriver from the wire; attaching a first nodule to the wire at the end nearest the patient's urethra; pulling the wire back up through the incision site until the first nodule lodges in the tip of the sound; pulling the tip of the sound out through the incision site by pulling the wire up through the incision site; removing the first nodule from the wire; placing a catheter on the wire; placing a second nodule on the wire; advancing the second nodule to the tip of the catheter by pulling the wire through the patient's urethra; pulling the catheter tip into the end of the sound from which the removable tip was removed by pulling the wire through the patient's urethra; wherein the catheter comprises a balloon, inflating the balloon; pulling the sound and catheter out through the patient's urethra; deflating the balloon; separating the catheter from the sound; pulling the catheter back into the patient's bladder; inflating the balloon; and attaching the catheter to drainage.

Also described are a knife-on-a-wire, a trocar-on-a-wire and a screwdriver-on-a-wire

### BRIEF DESCRIPTION OF THE DRAWINGS

Figure 1 is a perspective view of the urethral sound of the present invention.
Figure 2 is a section view of the urethral sound of the present invention.
Figure 3 is a perspective view of the urethral sound of the present invention with the tip removed.
Figure 4 is a partial schematic view of four different embodiments of the urethral sound of the present invention, illustrating different "throws" available.
Figure 5 is a section view of a urethral sound device inserted into the bladder of a patient. As compared to the sound shown in Figure 6, this sound has a relatively shorter throw and a relatively greater angle.
Figure 6 is a section view of the urethral sound of the present invention inserted into the bladder of a patient. As compared to the sound shown in Figure 5, this sound has a relatively longer throw and a relatively smaller angle.
Figure 7 is a perspective view of a Councill catheter.
Figure 8 is a perspective view of Councill catheter.
Figure 9 is a partial perspective view of the tip of a Councill catheter.
Figure 10 is a partial perspective view of the tip of a Councill catheter with the balloon inflated.
Figure 11 is a perspective view of the exemplary balloon capture embodiment with the sleeve disconnected from the sound.
Figure 12 is a perspective view of the exemplary balloon capture embodiment with the sleeve connected to the sound.
Figure 13 is a partial section view of the exemplary balloon capture embodiment in which the balloon is deflated.
Figure 14 is a partial section view of the exemplary balloon capture embodiment in which the balloon is inflated.
Figure 15 is a perspective view of the exemplary clamshell capture embodiment in which the clamshell is disengaged from the catheter and from the sound.
Figure 16 is a perspective view of the exemplary clamshell capture embodiment in which the clamshell is closed over the catheter but not attached to the sound.
Figure 17 is a perspective view of the exemplary clamshell capture embodiment in which the clamshell is closed over the catheter and attached to the sound.
Figure 18 is a section view of the exemplary clamshell and a partial section view of the catheter and sound.
Figure 19 is a section view of the exemplary clamshell when it is closed over the catheter and attached to the sound (as shown in Figure 17).
Figure 20 is a perspective view of the exemplary sleeve capture embodiment which the catheter is not inserted into the threaded extension of the sound, and the sleeve is not installed over the threaded extension of the sound.
Figure 20A is a section view of the exemplary removable tip of the sound for use with the sleeve capture embodiment.
Figure 21 is a perspective view of the exemplary sleeve capture embodiment in which the catheter is inserted into the threaded extension of the sound, but the sleeve is not installed over the threaded extension of the sound.
Figure 22 is a perspective view of the exemplary sleeve capture embodiment in which the catheter is inserted into the threaded extension of the sound, and the sleeve is installed over the threaded extension of the sound.
Figure 23 is a partial section view of the exemplary catheter inserted into the threaded extension of the sound and a section view of the sleeve positioned over the catheter but not over the threaded extension of the sound.
Figure 24 is a partial section view of the exemplary catheter inserted into the threaded extension of the sound and a section view of the sleeve positioned over the threaded extension of the sound.
Figure 25 is a section view of the urethral sound of the present invention inside the patient's bladder.
Figure 26 is a section view of the urethral sound of the present invention after it has passed through the patient's abdominal wall.
Figure 27 is a section view of the urethral sound of the present invention with a wire passed through the sound.
Figure 28 is a section view of the urethral sound of the present invention with a wire passed through the sound and a catheter on the wire.
Figure 29 is a partial perspective view of the ball and wire.
Figure 30 is a section view of the urethral sound of the present invention with a wire passed through the sound and a catheter and ball on the wire.
Figure 31 is a partial perspective view of the ball on the wire.
Figure 32 is a section view of the urethral sound of the present invention with a wire passed through the sound, a catheter and ball on the wire, and the ball pulled to the tip of the catheter.
Figure 33 is a section view of the catheter tip with the ball on the wire and inside the catheter tip.
Figure 34 is a section view of the urethral sound of the present invention with a wire passed through the sound, a catheter and ball on the wire, the ball pulled to the tip of the catheter, and the catheter tip pulled to the tip of the sound.
Figure 35 is a section view of the urethral sound of the present invention with a wire passed through the sound, a catheter and ball on the wire, the ball pulled to the tip of the catheter, the catheter tip pulled to the tip of the sound, and the catheter pulled into the bladder.
Figure 36 is a section view of the catheter on a wire with a hook.
Figure 37 is a section view of the catheter on a wire with the hook as it passes through the circular hole in the catheter tip.
Figure 38 is a section view of the hook after it has passed through the circular hole in the catheter tip and into the inside of the catheter.
Figure 39 is a section view of the hook on the wire as the wire is being pulled back through the sound.
Figure 40 is a section view of the knife on a wire.
Figures 40A and 40B are side views of two alternate embodiments of the knife. Figure 40C is a section view of the knife, showing the cut made by the knife blades.
Figure 41 is a section view of the knife on a wire with the tip of the knife in contact with the tip of the sound.
Figure 42 is a section view of the trocar on a wire.
Figure 42A is a section view of the trocar.
Figure 42B is a perspective view of the trocar.
Figure 42C is a section view of the trocar, showing the cut made by the trocar blades.
Figure 43 is a section view of the trocar on a wire with the tip of the trocar in contact with the tip of the sound.
Figures 44-93 show exemplary embodiments, not forming part of the present invention.

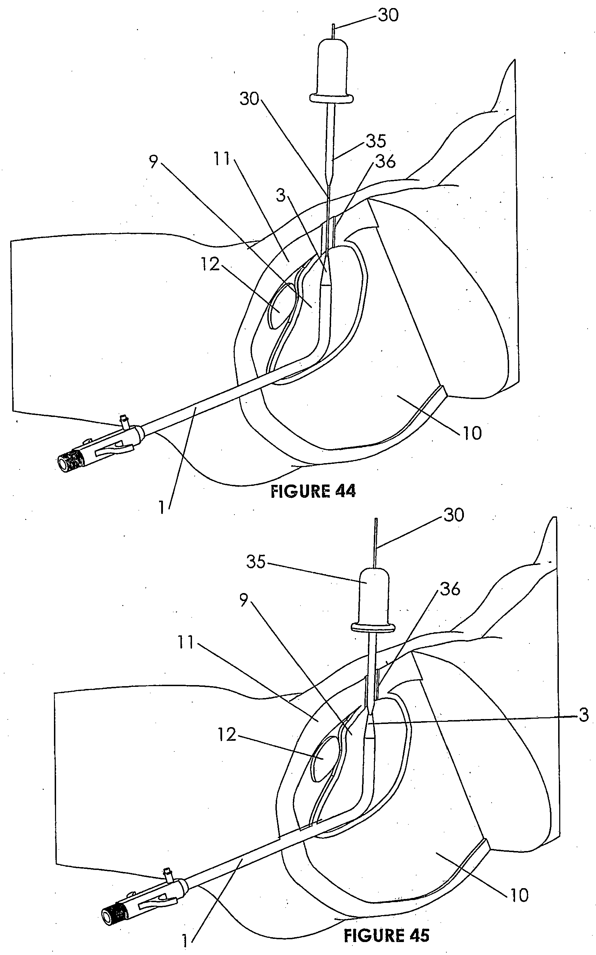

Figure 44 is a section view of the screwdriver on a wire.
Figure 45 is a section view of the screwdriver on a wire with the tip of the screwdriver in contact with the tip of the sound.

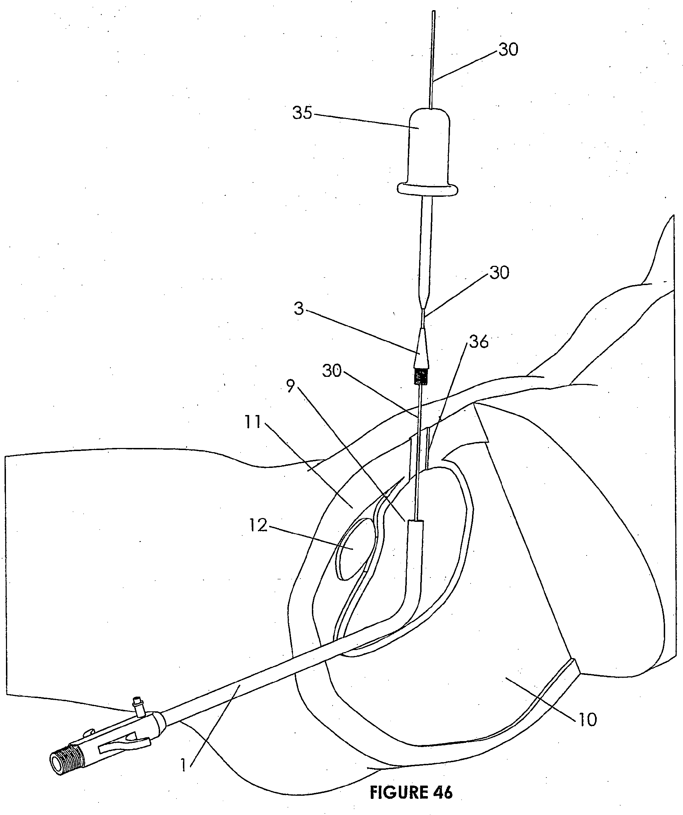

Figure 46 is a section view of the screwdriver on a wire after the tip of the sound has been unscrewed from the sound.

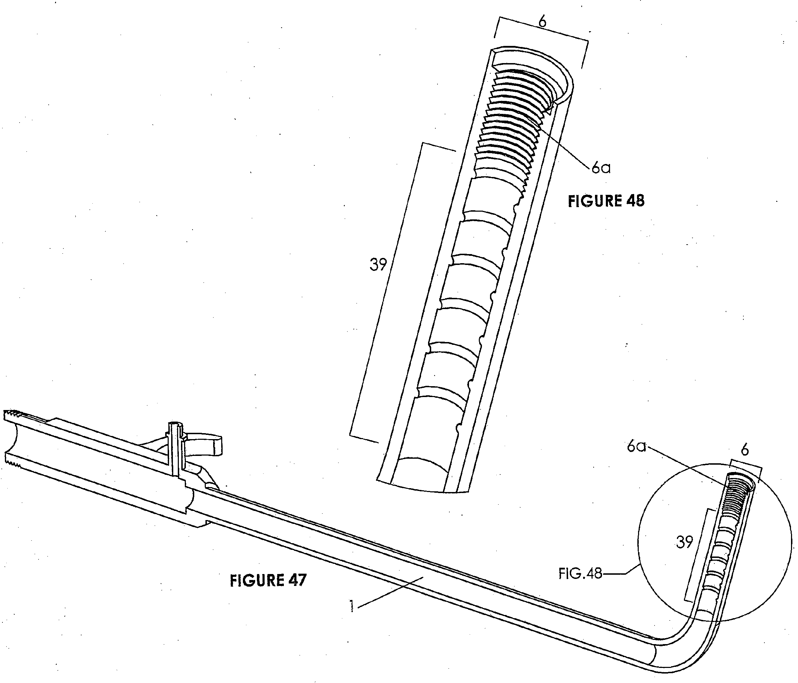

Figure 47 is a section view of a first embodiment of a sound that has been adapted to facilitate the balloon capture method.

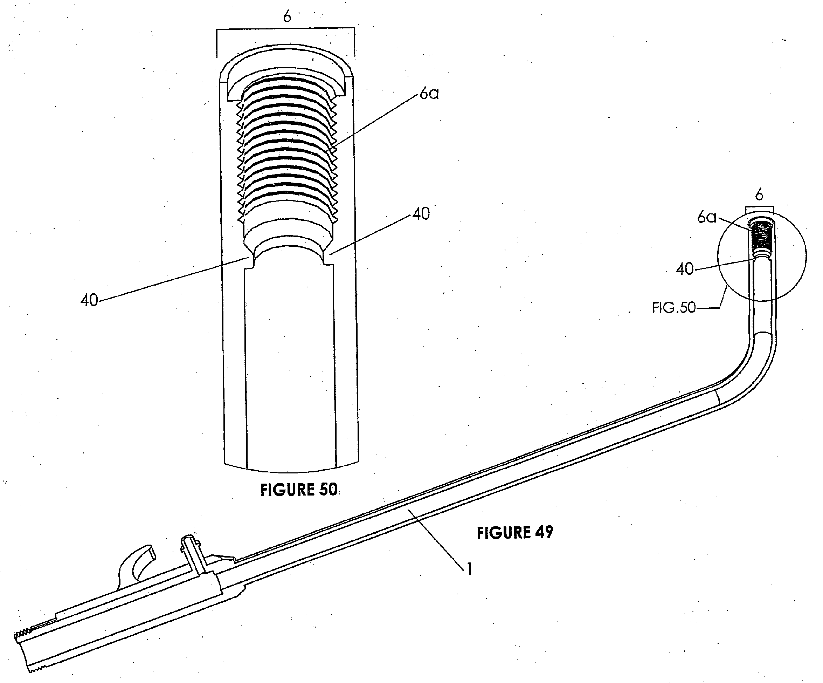

Figure 48 is a partial section view of the sound shown in Figure 47. Figure 49 is a section view of a second embodiment of a sound that has been adapted to facilitate the balloon capture method.
Figure 50 is a partial section view of the sound shown in Figure 49.

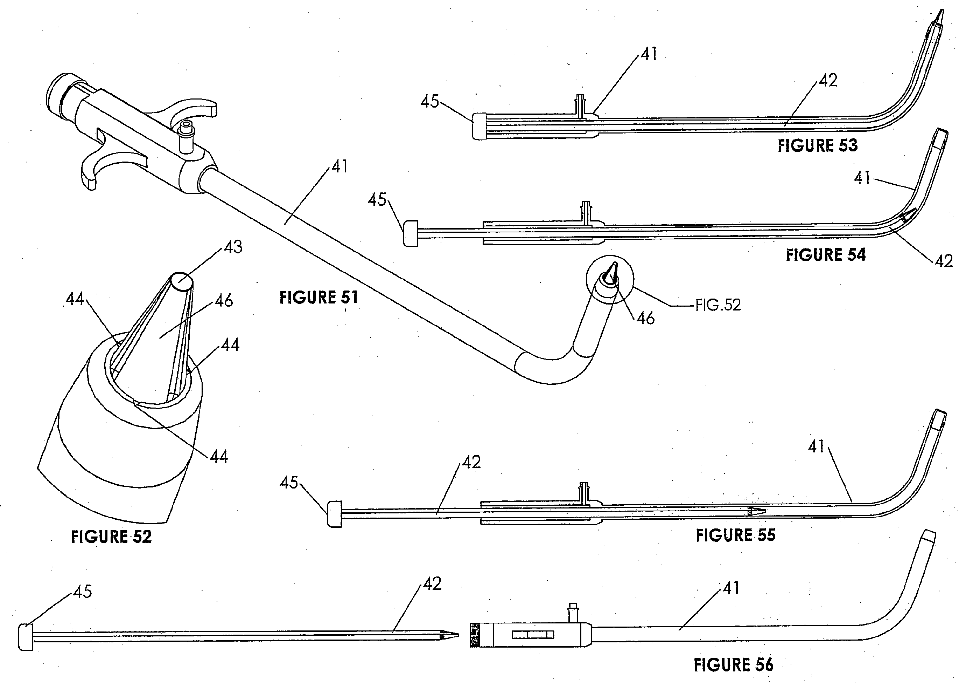

Figure 51 is a perspective view of a lipped sound with a first embodiment of an obturator.
Figure 52 is a partial perspective view of the tip of the lipped sound and obturator shown in Figure 51.
Figure 53 is a section view of the obturator of Figure 51 fully inserted inside the sound.
Figure 54 is a section view of the obturator of Figure 51 partially inserted inside the sound.
Figure 55 is a section view of the obturator of Figure 51 partially inserted inside the sound but more fully withdrawn than in Figure 54.
Figure 56 is a section view of the obturator of Figure 51 fully withdrawn from the sound.

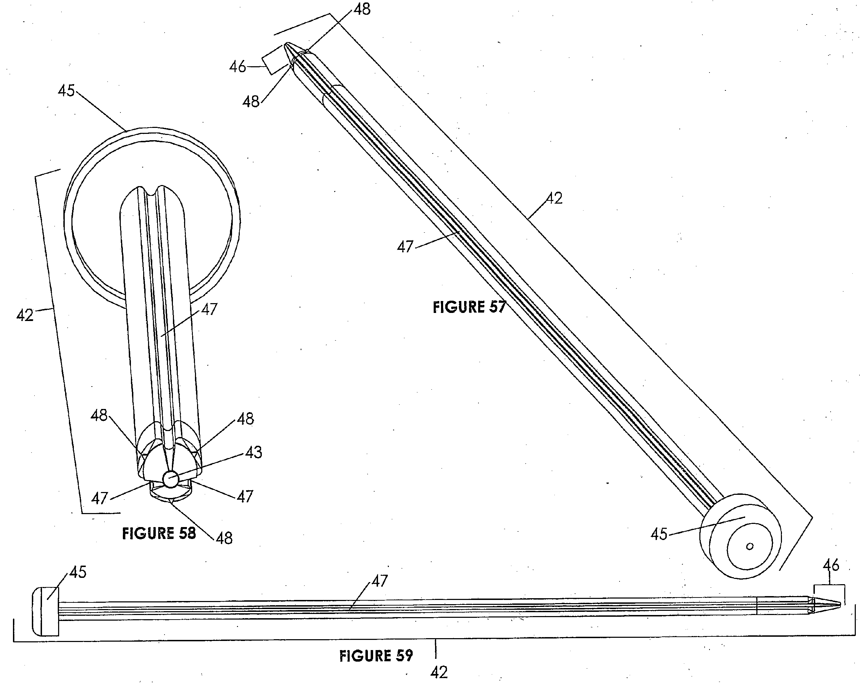

Figure 57 is a rear perspective view of the obturator of Figure 51.
Figure 58 is a front perspective view of the obturator of Figure 51. Figure 59 is a top view of the obturator of Figure 51.

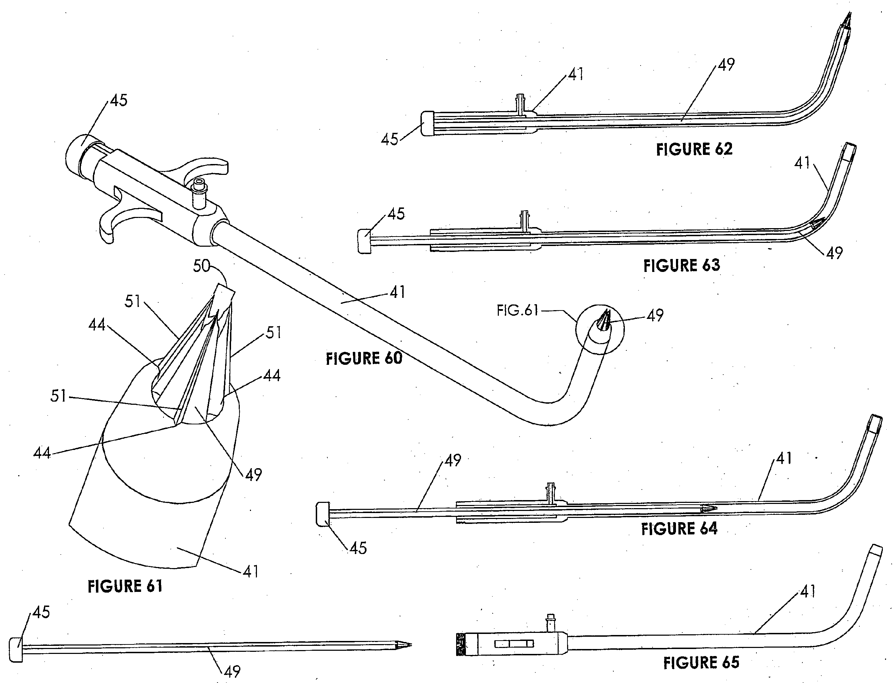

Figure 60 is a perspective view of a lipped sound with a first embodiment of a retractable trocar.
Figure 61 is a partial perspective view of the tip of the lipped sound and retractable trocar shown in Figure 60.
Figure 62 is a section view of the retractable trocar of Figure 60 fully inserted inside the sound.
Figure 63 is a section view of the retractable trocar of Figure 60 partially inserted inside the sound.
Figure 64 is a section view of the retractable trocar of Figure 60 partially inserted inside the sound but more fully withdrawn than in Figure 63.
Figure 65 is a section view of the retractable trocar of Figure 60 fully withdrawn from the sound.

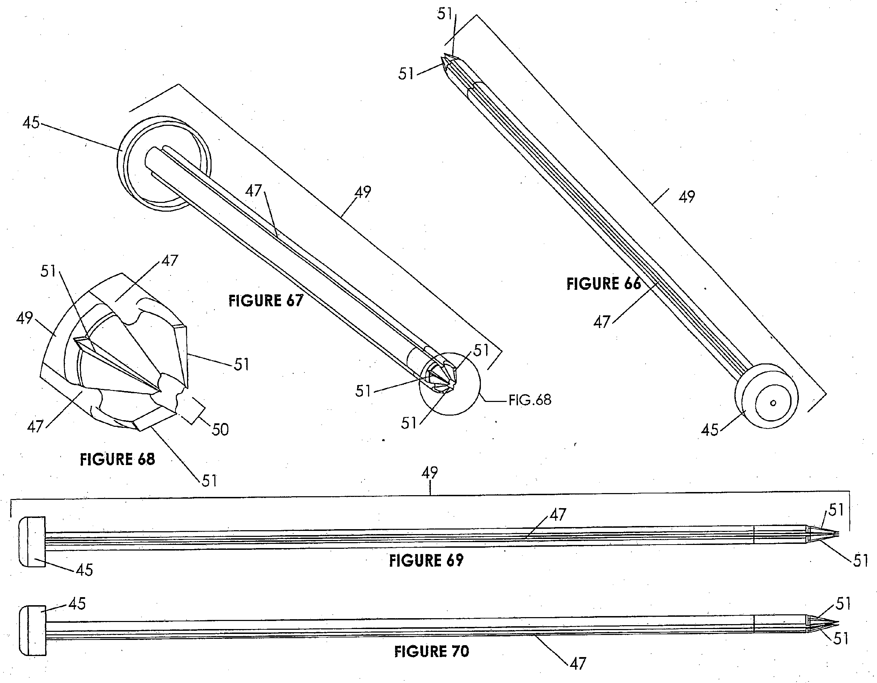

Figure 66 is a rear perspective view of the retractable trocar of Figure 60.
Figure 67 is a front perspective view of the retractable trocar of Figure 60.
Figure 68 is a partial perspective view of the tip of the retractable trocar of Figure 60.
Figure 69 is a top view of the retractable trocar of Figure 60.
Figure.70 is a side view of the retractable trocar of Figure 60.

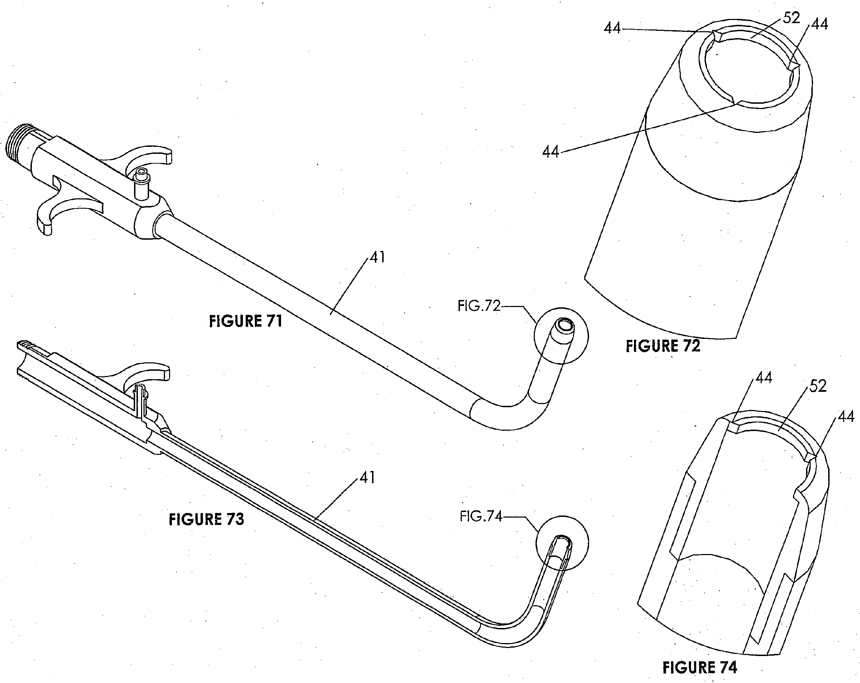

Figure 71 is a perspective view of the lipped sound of Figure 51.
Figure 72 is a partial perspective view of the tip of the lipped sound of Figure 51.
Figure 73 is a section view of the lipped sound of Figure 51.
Figure 74 is a partial section view of the tip of the lipped sound of Figure 51.

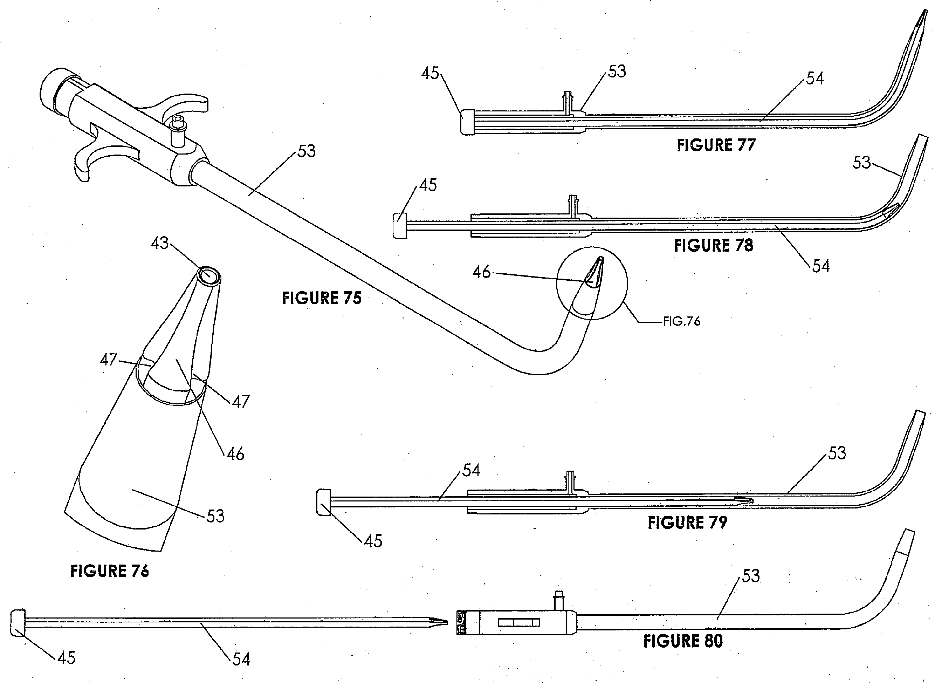

Figure 75 is a perspective view of a sound with a second embodiment of an obturator.
Figure 76 is a partial perspective view of the tip of the sound and obturator shown in Figure 75.
Figure 77 is a section view of the obturator of Figure 75 fully inserted inside the sound.
Figure 78 is a section view of the obturator of Figure 75 partially inserted inside the sound.
Figure 79 is a section view of the obturator of Figure 75 partially inserted inside the sound but more fully withdrawn than in Figure 78.
Figure 80 is a section view of the obturator of Figure 75 fully withdrawn from the sound.

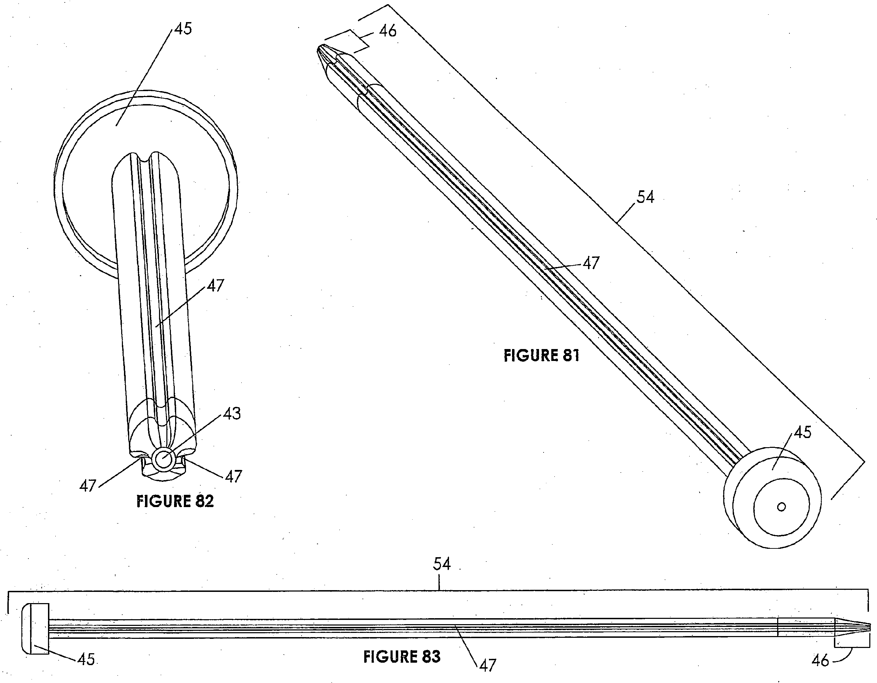

Figure 81 is a rear perspective view of the obturator of Figure 75.
Figure 82 is a front perspective view of the obturator of Figure 75.
Figure 83 is a top view of the obturator of Figure 75.

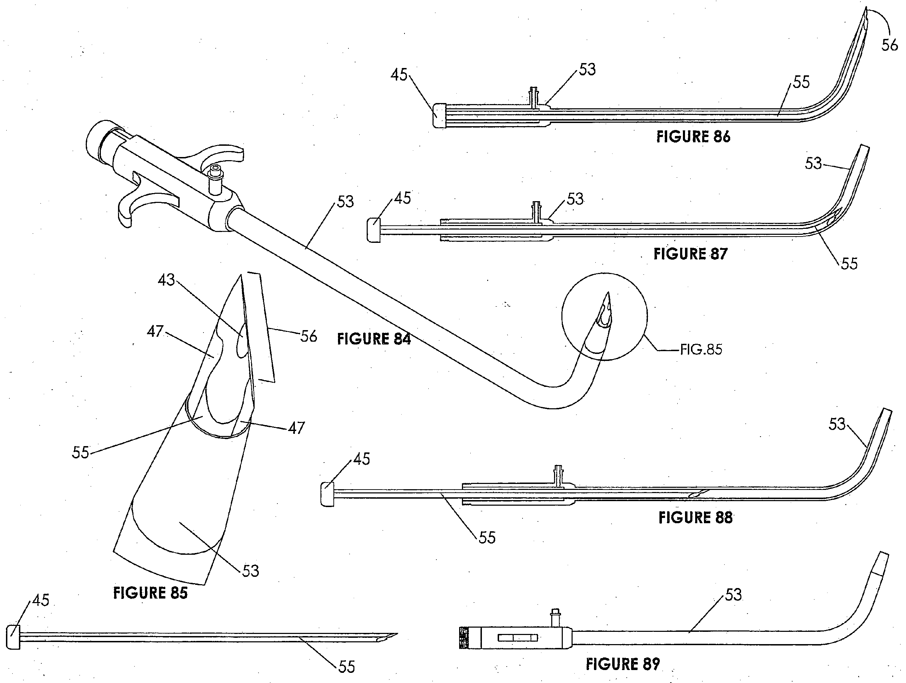

Figure 84 is a perspective view of a sound with a second embodiment of a retractable trocar.
Figure 85 is a partial perspective view of the tip of the sound and retractable trocar shown in Figure 84.
Figure 86 is a section view of the retractable trocar of Figure 84 fully inserted inside the sound.
Figure 87 is a section view of the retractable trocar of Figure 84 partially inserted inside the sound.
Figure 88 is a section view of the retractable trocar of Figure 84 partially inserted inside the sound but more fully withdrawn than in Figure 87.
Figure 89 is a section view of the retractable trocar of Figure 84 fully withdrawn from the sound.

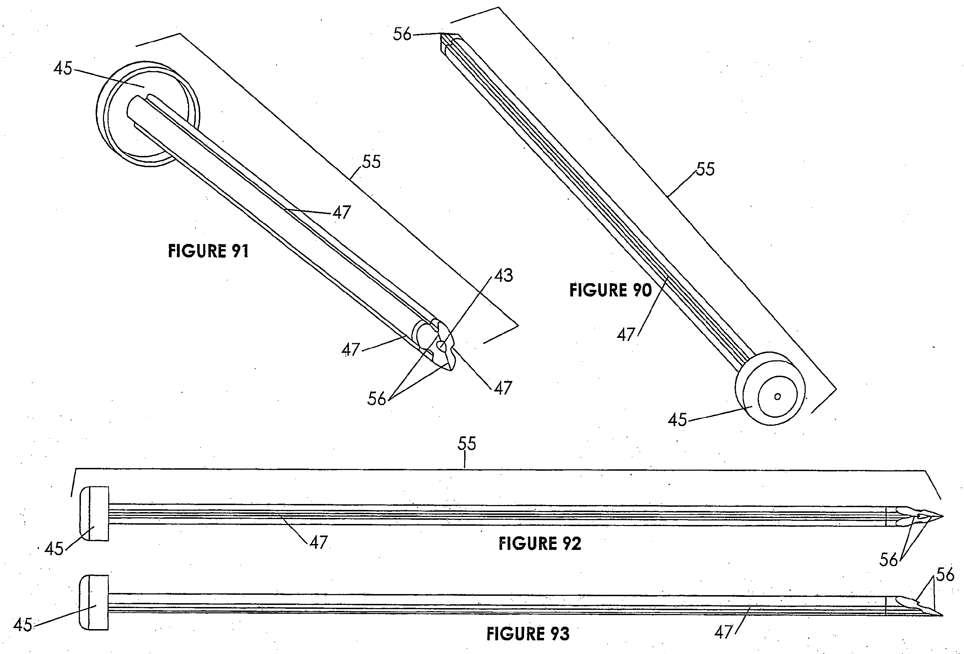

Figure 90 is a rear perspective View of the retractable trocar of Figure 84.
Figure 91 is a front perspective view of the retractable trocar of Figure 84.
Figure 92 is a top view of the retractable trocar of Figure 84.
Figure 93 is a side view of the retractable trocar of Figure 84.

### REFERENCE NUMBERS

- 1: Sound
- 2: Handle
- 3: Tip of sound (with threaded end)
- 4: Hollow channel
- 5: Threaded end of tip of sound
- 6: Distal end of sound
- 6a: Internal threads (sound)
- 7: Proximal end of sound
- 8: Sound with shorter throw and relatively greater angle
- 8a: Sound with longer throw and relatively smaller angle
- 9: Bladder
- 10: Peritoneal cavity
- 11: Abdominal wall
- 12: Pubic bone
- 13: Catheter shaft
- 14: Catheter tip
- 15: Balloon
- 16: Lateral holes (in catheter tip)
- 17: Inflation member
- 18: Drainage connection
- 19: Circular hole (in Councill catheter tip)
- 20: Sleeve (balloon capture embodiment)
- 21: Flange
- 22: Clamshell capture device
- 23: Pegs
- 24: Threaded end of clamshell capture device
- 25: Notch
- 26: Threaded extension (of sound)
- 27: Sleeve
- 28: Pin
- 29: Holes in threaded extension (of sound)
- 30: Wire
- 31: Ball
- 32: Hook
- 33: Knife
- 33a: Leading edge of knife
- 33b: Knife blades
- 33c: Threaded end of knife
- 34: Trocar
- 34a: Trocar blades
- 34b: Inner threads of trocar
- 35: Screwdriver
- 36: Incision site
- 37: Tip of sound (threaded on the inside)
- 38: Funnel
- 39: Internal ribs
- 40: Internal flange
- 41: Lipped sound
- 42: Obturator (first embodiment, for use with lipped sound)
- 43: Hole
- 44: Triangular indentations
- 45: Plug
- 46: Tapered end (of obturator)
- 47: Water channels
- 48: Wings
- 49: Retractable trocar (first embodiment, for use with lipped sound)
- 50: Cutting end
- 51: Blades
- 52: Lip
- 53: Sound (no lip)
- 54: Obturator (second embodiment, for use with non-lipped sound)
- 55: Retractable trocar (second embodiment, for use with non-lipped sound)
- 56: Angled cutting edge

### DETAILED DESCRIPTION OF INVENTION

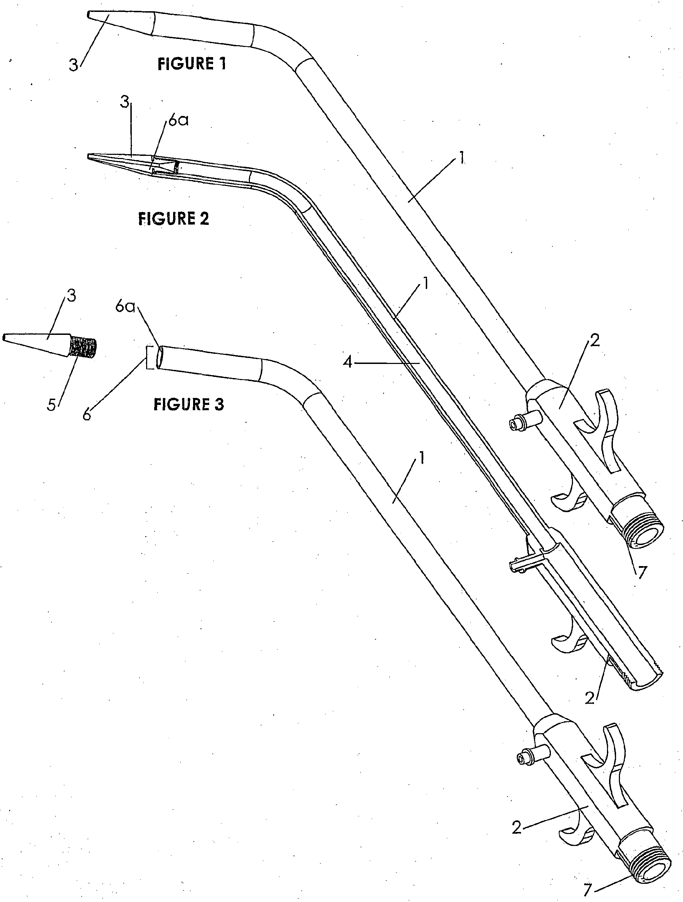

Figure 1 is a perspective view of the urethral sound of the present invention. This figure shows the sound 1, the handle 2, and the tip 3. The sound is hollow, and there are holes at either end of the sound for the insertion of a wire.

Figure 2 is a section view of the urethral sound of the present invention. This figure shows the sound 1, the handle 2, the tip 3, and the hollow channel 4, which extends from one end of the sound to the other.

Figure 3 is a perspective view of the urethral sound of the present invention with the tip removed. This figure shows the sound 1, the handle 2, and the tip 3. It also shows the threaded end 5 of the tip, which is inserted into the threaded distal end 6 of the sound. The proximal end of the sound 7 is also threaded for the addition of a Tuohy-Borst adapter or an endoscopic cap.

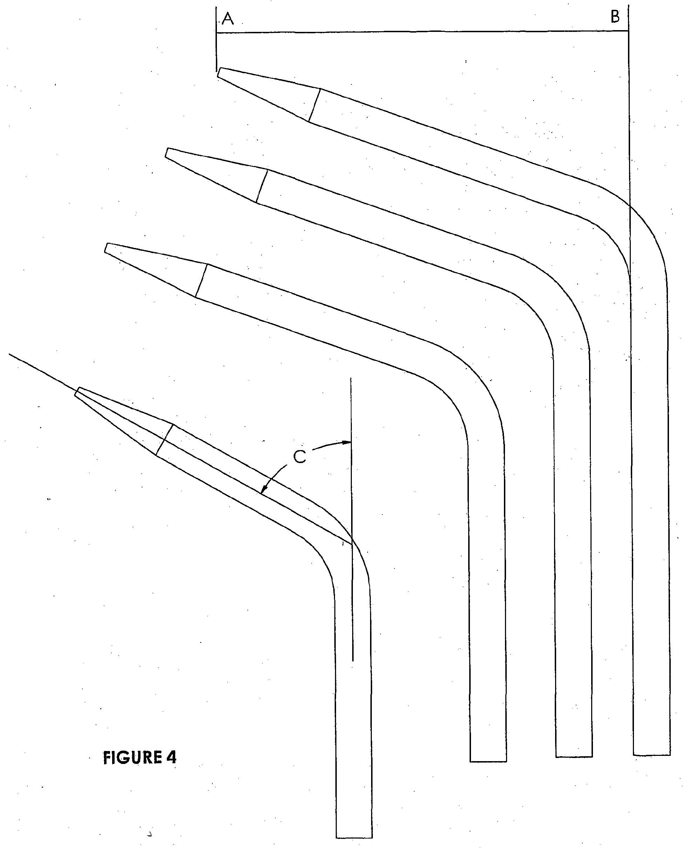

Figure 4 is a partial schematic view of four different embodiments of the urethral sound of the present invention, illustrating different "throws" available. The throw is defined in this figure as the distance from point A to point B. The sound of the present invention preferably has a throw that is at least six (6) centimeters long, although a shorter throw may be appropriate in certain situations. Different throw lengths may be useful for different types of patients (for example, male or female, adult or child). In this figure, the throw length increases from the sound shown at the bottom to the sound shown at the top. In practice, the throw will probably not be longer than fourteen (14) centimeters. The angle of the sound is shown as C. The angle of the sound is preferably in the range of sixty (60) to eighty (80) degrees. The angle of all of the sounds shown in this figure is seventy (70) degrees.

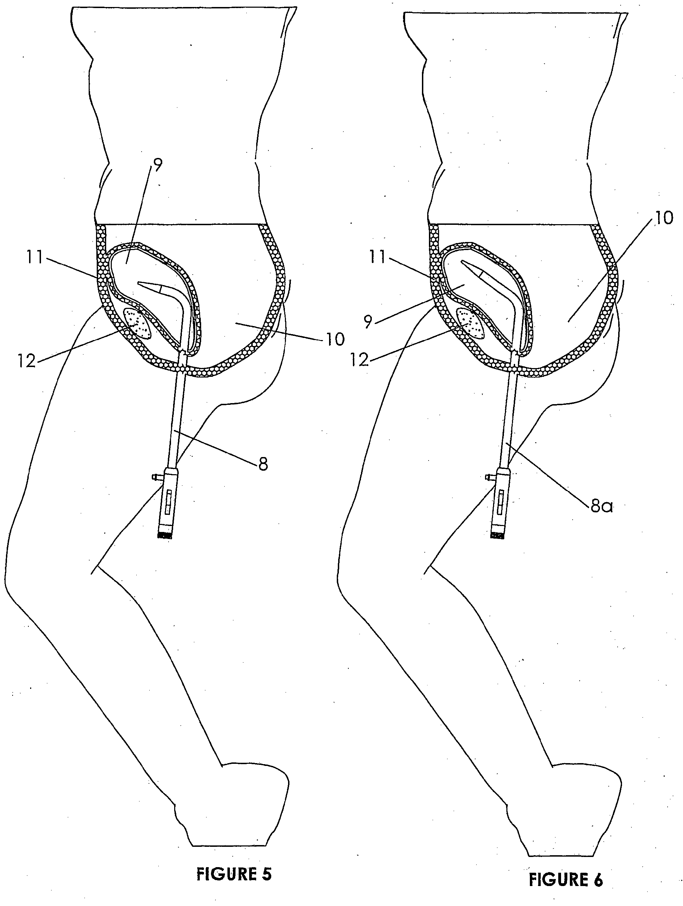

Figures 5 and 6 illustrate the difference between a sound with a shorter throw and relatively greater angle (80 degrees) (Figure 5) and a sound with a longer throw and relatively smaller angle (60 degrees) (Figure 6).

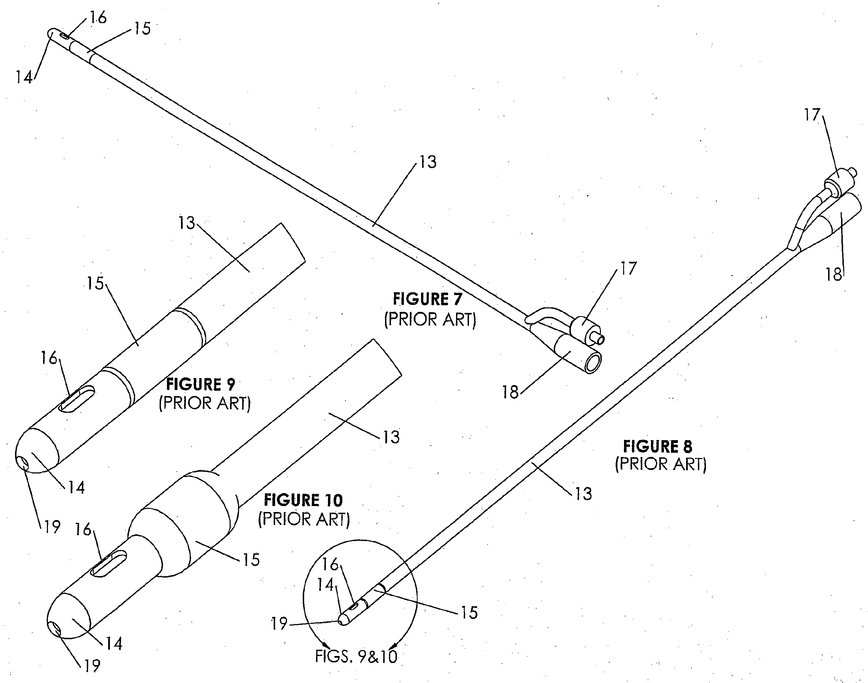

Figure 7 is a perspective view of a Councill catheter. Although the present invention can be used with many different types of catheters and is not limited to the Councill catheter (except for the ball-on-a-wire, hook-on-a-wire, nodule-on-a-wire and crimped wire embodiments), a drawing of the Councill catheter is provided for illustrative purposes. This figure shows the catheter shaft, 13, the catheter tip 14, one of two lateral holes 16 in the catheter tip 14, and the uninflated balloon 15. On the proximal end of the catheter, the inflation member 17 and drainage connection 18 are also shown. The purpose of the inflation member 17 is to inflate the balloon 15 on the distal end of the catheter.

Figure 8 is a perspective view of Councill catheter. As in Figure 7, this figure shows the catheter shaft 13, the catheter tip 14, one of two lateral holes 16 in the catheter tip 14, and the balloon 15. It also shows a circular hole 19 at the end of the catheter tip 14. A Foley catheter is the same as the Councill catheter, except that it does not have this circular hole 19 in the catheter tip. In addition, this figure shows the inflation member 17 and drainage connection 18. The circle at the distal end of the catheter shows the orientation of Figures 9 and 10.

Figure 9 is a partial perspective view of the tip of a Councill catheter. This figure shows the distal end of the catheter only, with the tip 14, balloon 15, one of two lateral holes 16, and the circular hole 19 in the catheter tip. Figure 10 is a partial perspective view of the tip of a Councill catheter with the balloon 15 inflated.

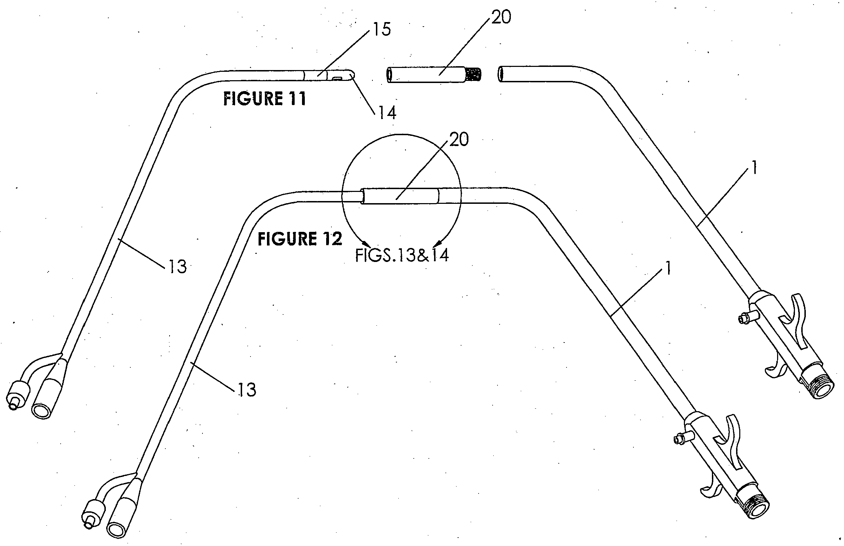

Figures 11-14 illustrate the exemplary balloon capture embodiment. Figure 11 is a perspective view of the balloon capture embodiment with the sleeve disconnected from the sound. This figure shows the sound 1, the catheter 13, the catheter tip 14, and the balloon 15. It also shows a sleeve 20, which is threaded at one end so that it can be inserted into the threaded distal end of the sound, where the tip (not shown) would normally attach.

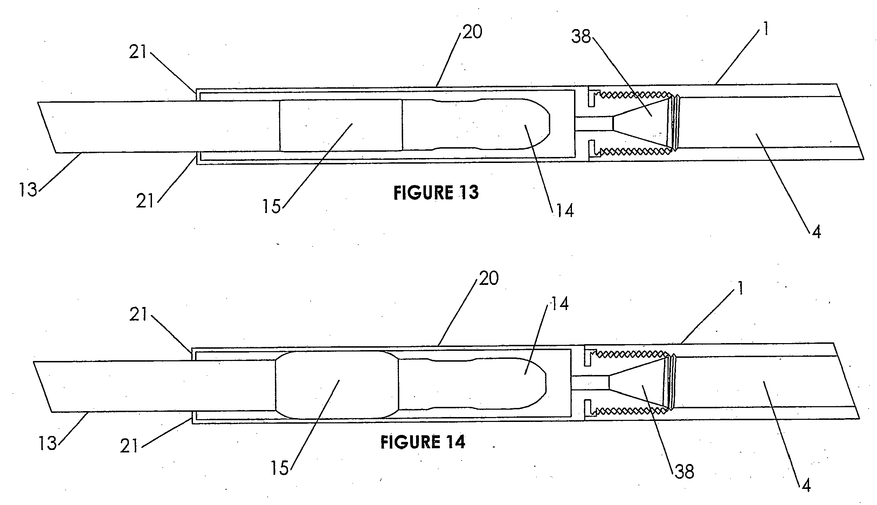

Figure 12 is a perspective view of the exemplary balloon capture embodiment with the sleeve connected to the sound. This figure shows the catheter tip and balloon (not shown) inserted into the sleeve 20. The circle around the sleeve 20 shows the orientation of Figures 13 and 14.

Figure 13 is a partial section view of the exemplary balloon capture embodiment in which the balloon is deflated. This figure shows the catheter tip 14 and balloon 15 inserted into the sleeve 20. It also shows a flange 21 that is integral to the sleeve 20 and that extends inward at the distal end of the sleeve. The purpose of the flange is to keep the catheter in place when the balloon is inflated, although in practice the friction created by the inflated balloon against the inner walls of the sleeve is sufficient to keep the catheter in place. Figure 14 is a partial section view of the exemplary balloon capture embodiment in which the balloon 15 is inflated. Both of these figures show the optional funnel 38 inside the threaded end 5 of the removable tip 3. The funnel 38 is intended to facilitate passage of a wire (used in connection with other exemplary embodiments described below) through the end of the sound and out the removable tip 3 and to prevent it from getting hung up in the tip of the sound.

An alternate exemplary balloon capture method (not shown) does not utilize a sleeve or pin. In this method, the sound is inserted through the patient's urethra into the bladder and out the abdominal wall, and the tip of the sound is removed. The catheter tip with the balloon is then inserted into the end of the sound from which the removable tip was removed, and the balloon is inflated. Inflation of the balloon while inside the sound creates sufficient friction to hold the catheter in place. The catheter and sound are then pulled out through the patient's urethra, and the balloon is deflated. The catheter and sound are separated, and the catheter is pulled back up into the patient's bladder. The balloon is re-inflated, and the catheter is attached to drainage.

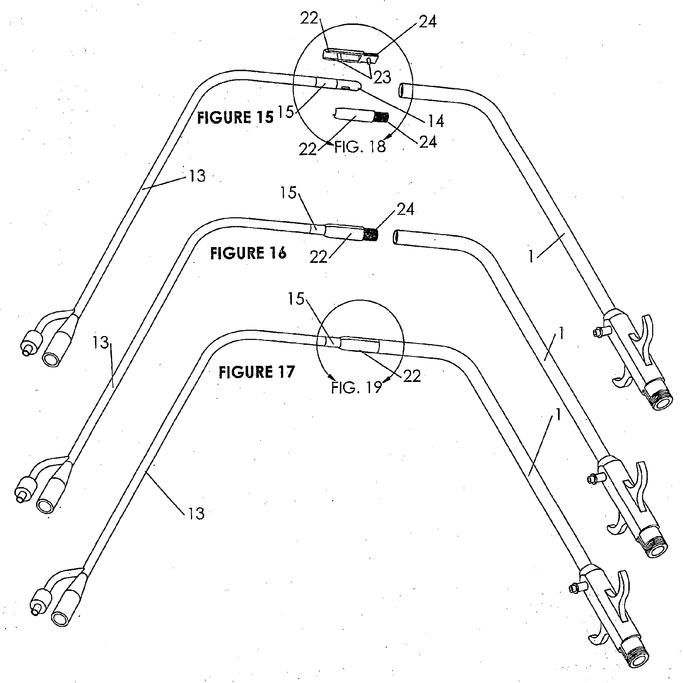

Figures 15-19 illustrate the exemplary clamshell capture embodiment. Figure 15 is a perspective view of the exemplary clamshell capture embodiment in which the clamshell is disengaged from the catheter and from the sound. This figure shows the catheter 13 and sound 1 with the tip (not shown) of the sound removed. It also shows a clamshell capture device 22 that comprises a top half and a bottom half. The top half of the clamshell capture device 23 comprises two pegs. One peg 23 passes through the lateral holes 16 in the catheter tip 14. The other peg 23 fits into a notch (not shown) in the bottom half of the clamshell capture device 23. The clamshell capture device has a threaded end 24 that fits into the threaded distal end of the sound.

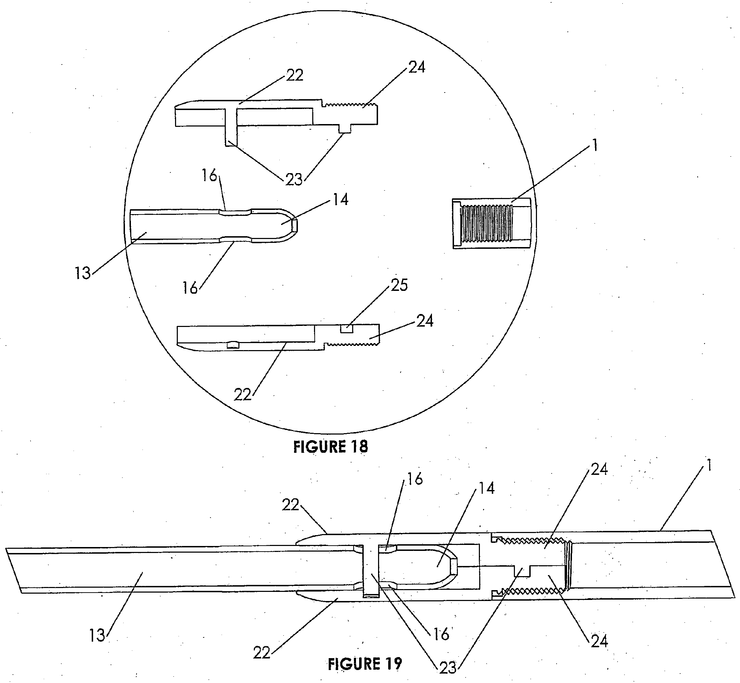

Figure 16 is a perspective view of the exemplary clamshell capture embodiment in which the clamshell 22 is closed over the catheter 13 but not attached to the sound 1. Figure 17 is a perspective view of the exemplary clamshell capture embodiment in which the clamshell 22 is closed over the catheter 13 and attached to the sound 1. The circle around the clamshell 22 shows the orientation of Figures 18 and 19. Although the balloon 15 is shown in Figures 15-17, it is shown only for orientation purposes and is not necessary for this embodiment to function.

Figure 18 is a section view of the exemplary clamshell and a partial section view of the catheter and sound. This figure shows the top and bottom halves of the clamshell 22 in relation to the catheter 13 and the sound 1. The top half of the clamshell comprises pegs 23, one of which passes through the lateral holes 16 in the catheter tip 14, and the other of which fits into a notch 25 in the threaded end 24 of the clamshell 22. Figure 19 is a section view of the clamshell when it is closed over the catheter and attached to the sound (as shown in Figure 17).

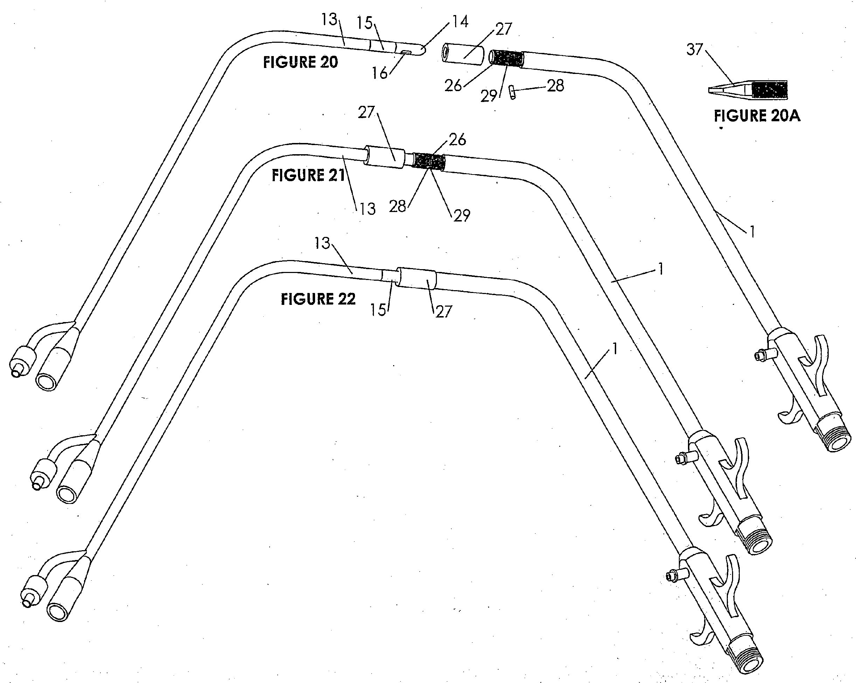

Figures 20-24 illustrate the exemplary sleeve capture embodiment. Figure 20 is a perspective view of the exemplary sleeve capture embodiment in which the catheter is not inserted into the threaded extension of the sound, and the sleeve is not installed over the threaded extension of the sound. This figure shows the catheter 13, the sound 1, and a threaded extension 26 on the distal end of the sound. This figure also shows the sleeve 27 and pin 28. As shown in subsequent figures, the sleeve 27 is first placed over the catheter tip 14 and slid past the balloon 15, the catheter tip 14 is then inserted into the threaded extension 26 of the sound 1, and the pin 28 is inserted through the lateral holes 16 in the catheter tip 14 and through two holes 29 (only one of which is shown in Figures 20 and 21) the threaded extension 26. Lastly, the sleeve 27, which is threaded on the inside, is screwed onto and over the threaded extension 26.

Figure 20A is a section view of the exemplary removable tip of the sound for use with the sleeve capture embodiment. As shown in this figure, in order to accommodate the threaded extension 26 of the sound 1, the removable tip 37 of the sound is preferably longer than the removable tip shown in Figure 3, and it is also threaded on the inside.

Figure 21 is a perspective view of the exemplary sleeve capture embodiment in which the catheter 13 is inserted into the threaded extension 26 of the sound 1, but the sleeve 27 is not installed over the threaded extension 26 of the sound 1. Figure 22 is a perspective view of the exemplary sleeve capture embodiment in which the catheter 13 is inserted into the threaded extension 26 of the sound 1, and the sleeve 27 is installed over the threaded extension 26 of the sound 1. The balloon 15 is shown in Figures 20 and 22 for orientation purposes but is not necessary for this embodiment to function.

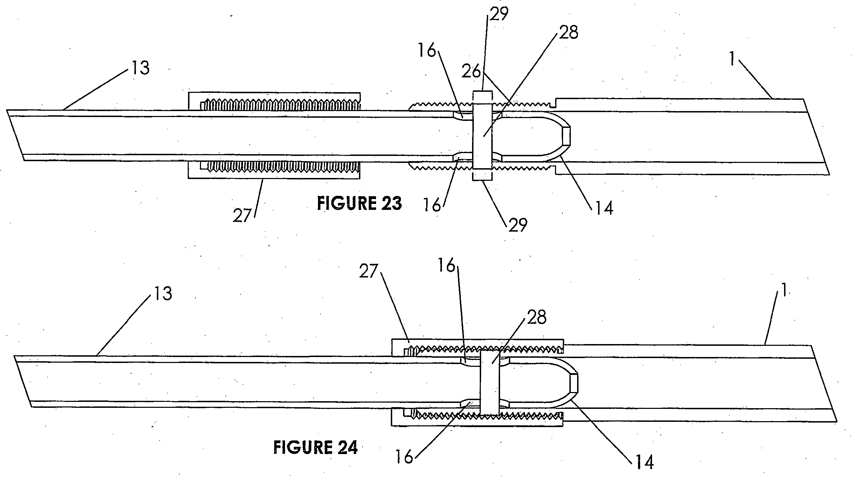

Figure 23 is a partial section view of the exemplary catheter inserted into the threaded extension of the sound and a section view of the sleeve positioned over the catheter but not over the threaded extension of the sound. This figure shows the catheter 13, the sound 1, and the sleeve 27 with inner threads. It also shows the catheter tip 14, the lateral holes 16 in the catheter tip 14, the threaded extension 26, and the holes 29 in the threaded extension. It also shows the pin 28 extending through the two lateral holes 26 in the catheter tip 14 and the two holes 29 in the threaded extension. Figure 24 is a partial section view of the exemplary catheter tip 14 inserted into the threaded extension 26 of the sound 1 and a section view of the sleeve 27 positioned over the threaded extension 26 of the sound.

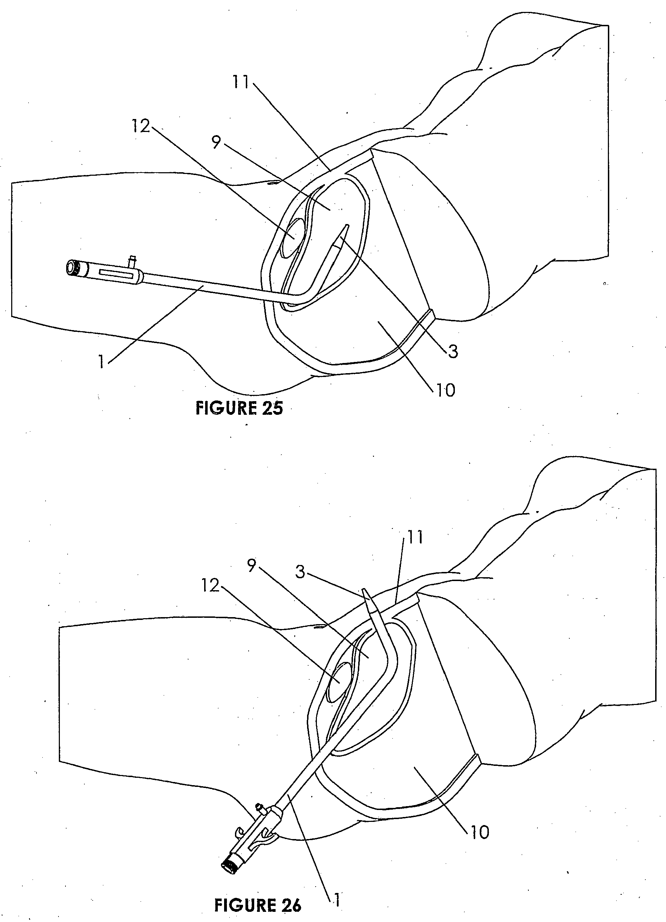

Figures 25-35 illustrate the ball-on-a-wire embodiment of the present invention. In this embodiment, a wire is passed through the urethral sound and inside the catheter, a ball is attached to the wire, the ball is pulled to the tip of the catheter, and the ball is used to guide the catheter to the tip of the sound.

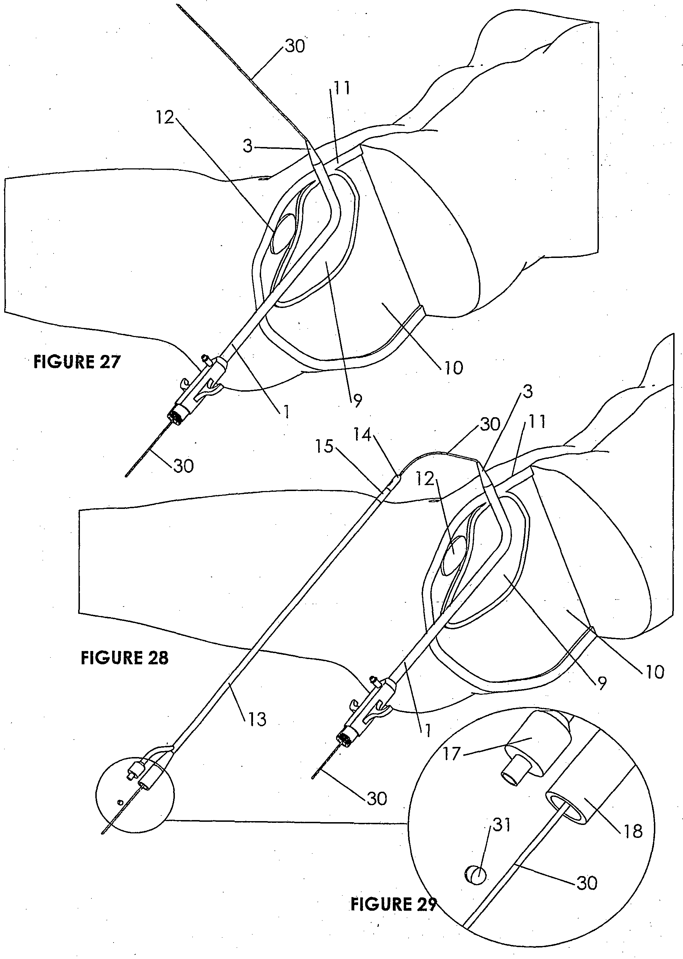

Figure 25 is a section view of the urethral sound 1 of the present invention inside the patient's bladder 9. Figure 26 is a section view of the urethral sound 1 of the present invention after it has passed through the patient's abdominal wall 11. Figure 27 is a section view of the urethral sound 1 of the present invention with a wire 30 passed through the sound. In practice, the wire will most likely be passed through the sound and held at the tip of the sound (so that it does not extend beyond the tip of the sound) as the sound is passed through the patient's urethra and bladder. The wire may then be advanced through the abdominal wall and act as a "guidewire" for the sound. Alternatively, the wire could be inserted into the sound after it has passed through the patient's urethra and bladder and out the abdominal wall. The wire is preferably similar to the Lunderquist-Ring torque guidewires or the Amplatz super-stiff guidewires, and the thickness of the wire may vary from approximately 0.10 to 0.20 centimeters. The distal end of the wire may be treated with polyvinyl pyrrolidine or another suitable substance so that it slides easily through the sound and through the urethra, bladder and abdominal wall.

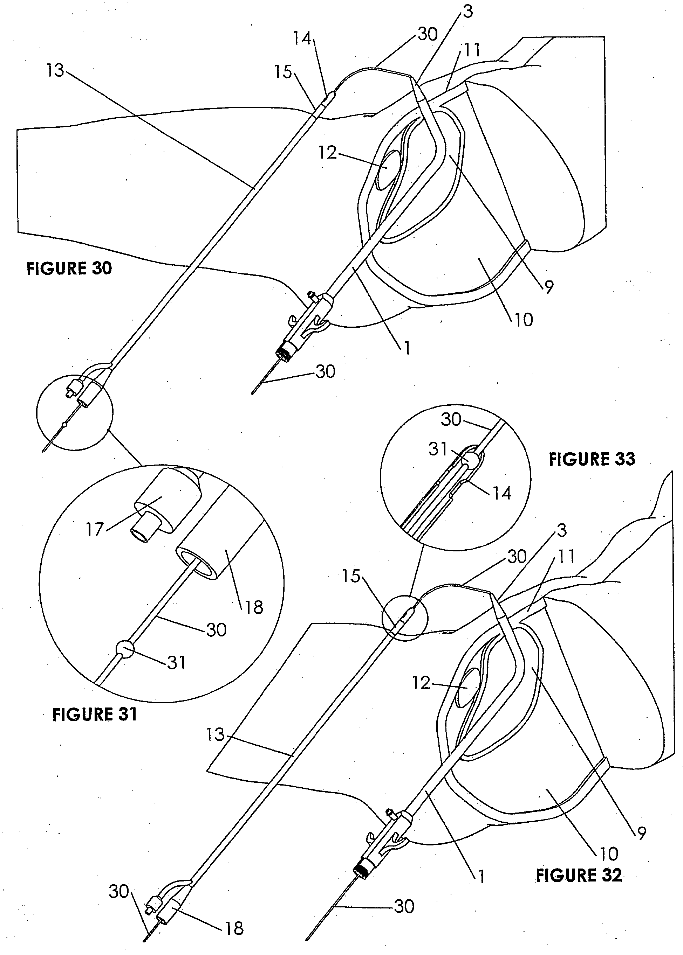

Figure 28 is a section view of the urethral sound 1 of the present invention with a wire 30 passed through the sound and a catheter 13 on the wire. At this stage in the procedure, enough wire is pushed through the sound so that the catheter can be placed on the wire. Figure 29 is a partial perspective view of the ball 31 and wire 30. After the wire is passed completely through the catheter, a ball 31 is placed on the wire. The ball can be attached to the wire in a number of different ways. For example, the wire may be serrated, and a ball with matching grooves may be crimped over the serrations in the wire. Alternatively, the wire may have a small threaded section, the ball may be threaded on the inside, and the ball may be screwed over the threaded section of the wire so that it passes over the threaded section and onto the smooth portion of the wire beyond the threads. The ball with then move freely up and down the wire on only one side of the threaded section (because the threads only allow the ball to move in one direction) and will not be able to pass beyond the threaded section when the wire is being used to pull the catheter into the bladder. Although a ball is shown for illustrative purposes, any hook, nodule or similar object could be used. Figure 30 is a section view of the urethral sound 1 of the present invention with a wire 30 passed through the sound and a catheter 13 and ball 31 on the wire. Figure 31 is a partial perspective view of the ball 31 on the wire 30.

Next, the wire 30 is pulled back through the sound 1 until the ball 31 lodges in the tip 14 of the catheter 13. Figure 32 is a section view of the urethral sound 1 of the present invention with a wire 30 passed through the sound, a catheter 13 and ball 31 on the wire, and the ball 31 pulled to the tit 14 of the catheter. Figure 33 is a section view of the catheter tip 14 with the ball 31 on the wire 30 and inside the catheter tip.

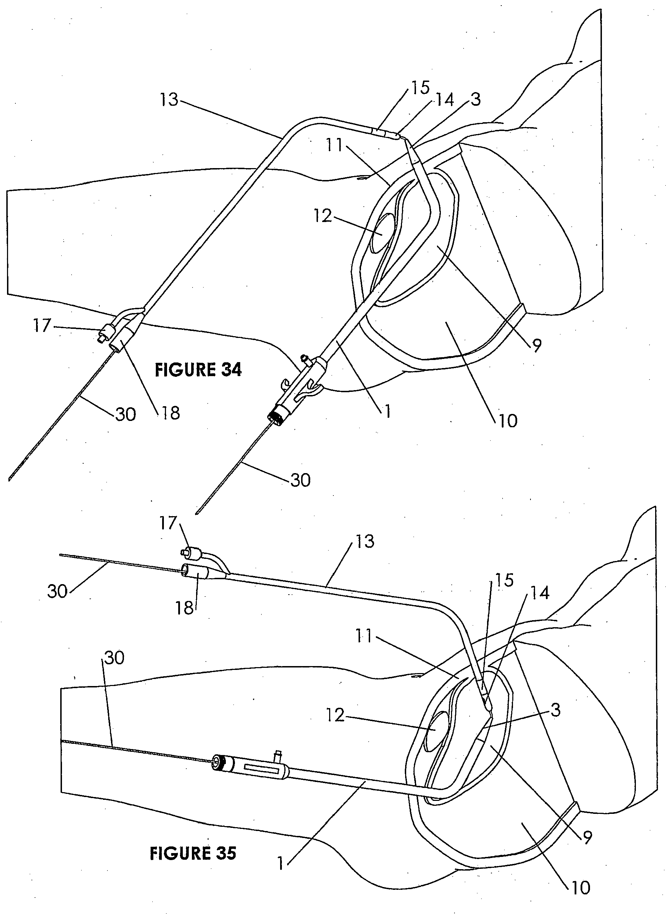

Next, the wire is pulled back through the sound 1 until the catheter tip 14 comes into contact with the tip 3 of the sound. Figure 34 is a section view of the urethral sound 1 of the present invention with a wire 30 passed through the sound, a catheter 13 and ball 31 on the wire, the ball 31 pulled to the tip 14 of the catheter, and the catheter tip 14 pulled to the tip 3 of the sound. Next, the sound 1 is retracted (pulled inside the patient's body and into the bladder), and the catheter 13 is guided by the ball 31 on the wire 30 into the patient's bladder 9, with the catheter tip 14 in contact with the tip 3 of the sound.

Figure 35 is a section view of the urethral sound 1 of the present invention with a wire 30 passed through the sound, a catheter 13 and ball 31 on the wire 30, the ball 31 pulled to the tip 14 of the catheter, the catheter tip 14 pulled to the tip 3 of the sound, and the catheter 13 pulled into the bladder 9. Once the catheter is in the desired position inside the bladder, the sound 1 is completely withdrawn through the patient's urethra, and the wire 30 is removed by pulling it out through the proximal end of the catheter (the end with the inflation member 17 and drainage connection 18). The balloon 15 is then inflated to keep the catheter in place inside the bladder 9.

Although the sound is shown in Figures 27-28, 30, 32, and 34-35 with the removable tip, in practice it may be desirable to remove the tip of the sound before the catheter is placed on the wire. That way, the catheter tip and balloon may be inserted into the end of the sound from which the removable tip was removed, and the balloon may be inflated inside the rigid walls of the sound, thereby providing additional stability for the catheter as it is pulled into the patient's bladder.

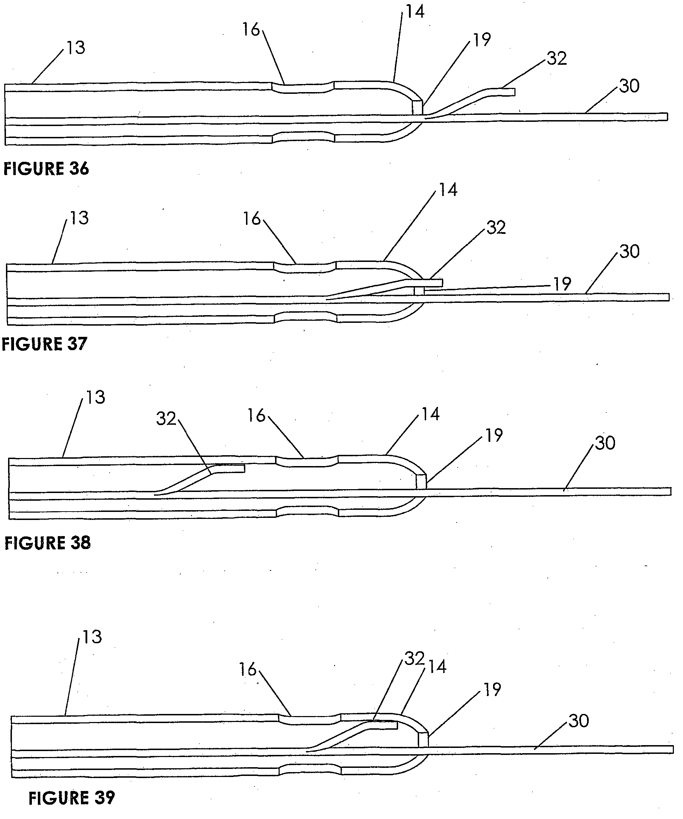

Figures 36-39 illustrate an alternate exemplary method of capturing the catheter on the wire. The procedure is the same as depicted in Figures 25-35, but a hook is used instead of a ball. Figure 36 is a section view of the catheter 13 on a wire 30 with a hook 32. Figure 36 illustrates one possible hook shape, but the present invention is not limited to any particular hook shape. If this particular type of hook is used, then it can be present on the wire before the wire is inserted through the sound and into the catheter. With other hook designs, the hook may need to be attached to the wire after the wire has passed through the sound and through the catheter, as discussed in connection with the ball-on-a-wire embodiment.

Figure 37 is a section view of the catheter 13 on a wire 30 with the hook 32 as it passes through the circular hole 19 in the catheter tip 14. Figure 38 is a section view of the hook 32 after it has passed through the circular hole 19 and into the inside of the catheter 13. Figure 39 is a section view of the hook 32 on the wire 30 as the wire is being pulled back through the sound (not shown). The hook 32 lodges in the catheter tip 14, thereby capturing the catheter so that it can be pulled toward the tip of the sound (not shown) and into the patient's bladder.

In yet another variation of the ball-on-a-wire and hook-on-a-wire embodiments, the wire can simply be crimped after the catheter is placed on the wire, such that the crimp serves the same purpose as the ball or hook. ,

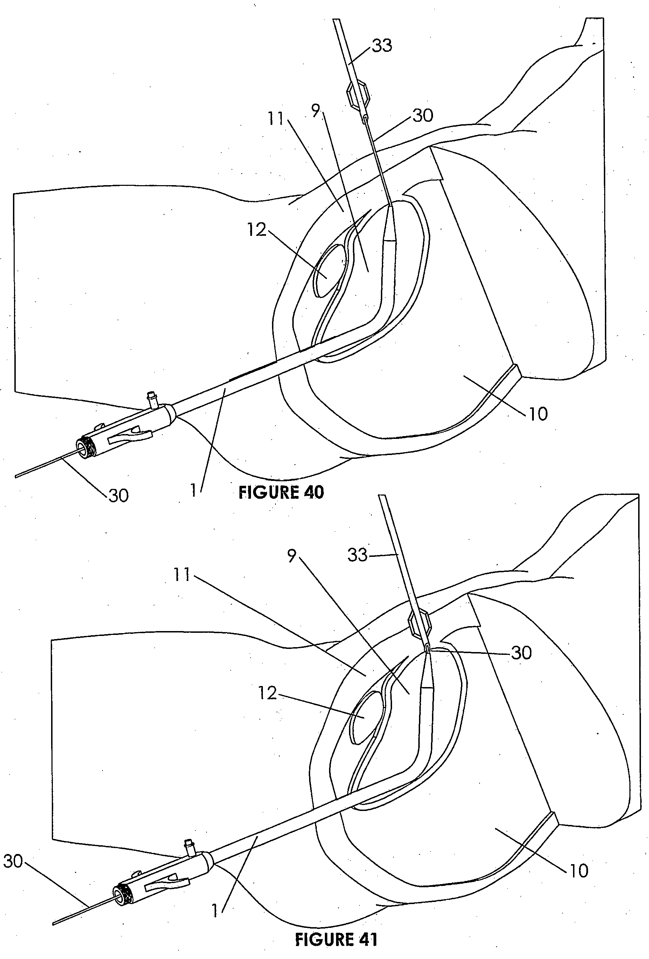

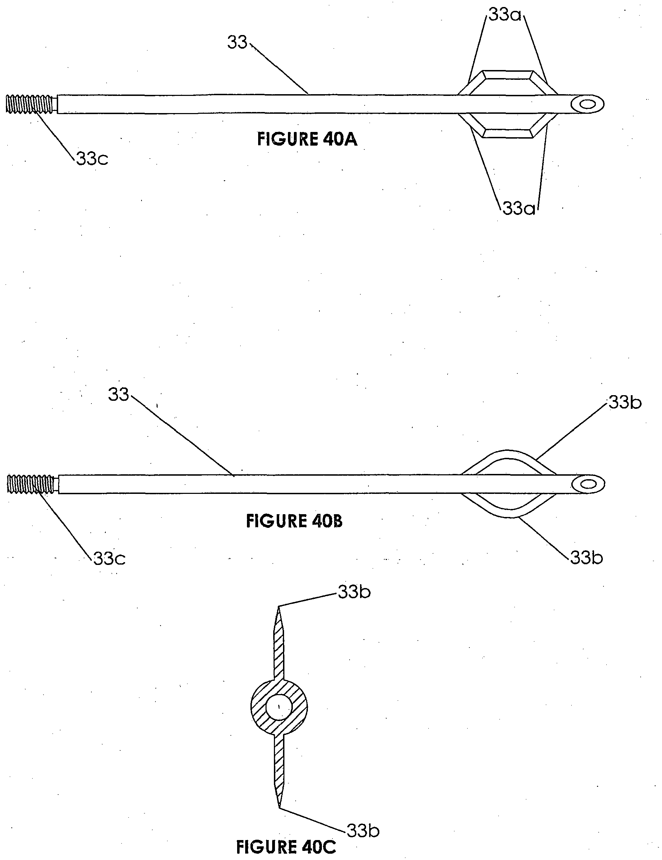

Figures 40-43 illustrate a number of different cutting tools that can be used in connection with the ball-on-a-wire (or hook-on-a-wire) procedure when the patient is so obese that the physician cannot get the tip of the sound to pass through the abdominal wall from the inside-out. Figure 40 shows a knife 33 on the wire 30. In this procedure, the knife 33 is placed onto the wire 30 after the wire is passed out through the patient's abdomen. The knife 33 is then guided by the wire 30 as it is pushed through the abdominal wall 11. When the knife 33 comes into contact with the tip 3 of the sound, as shown in Figure 41, the sound 1 is pulled back slightly and the knife 33 is pushed down again until it comes into contact with the tip 3 of the sound. This procedure ensures a uniform cut where the tip of the sound was when the knife first came into contact with it. The knife 33 is then removed from the wire 30, and the catheter is placed onto the wire and guided into the bladder using one of the exemplary methods described above (Figures 25-39).

Figures 40A and 40B are side views of two alternate embodiments of the knife. As shown in these figures, the knife 33 comprises a leading edge 33a, which is sharp, and a threaded end 33c. The threaded end allows an extension to be added to the knife for extra length. The knife also comprises blades 33b, which may be shaped either as shown in Figure 40A or Figure 40B. In the preferred embodiment, there are two knife blades 33b, which are oriented so that there is a 180-degree spacing between them. In figure 40A, the knife blades 33b form a hexagon shape, whereas in Figure 40B, they form a diamond shape. Figure 40C is a section view of the knife, showing the cut made by the knife blades 33b. Other than in claims 32 and 33, the present invention is not limited to any particular shape of the knife or knife blades.

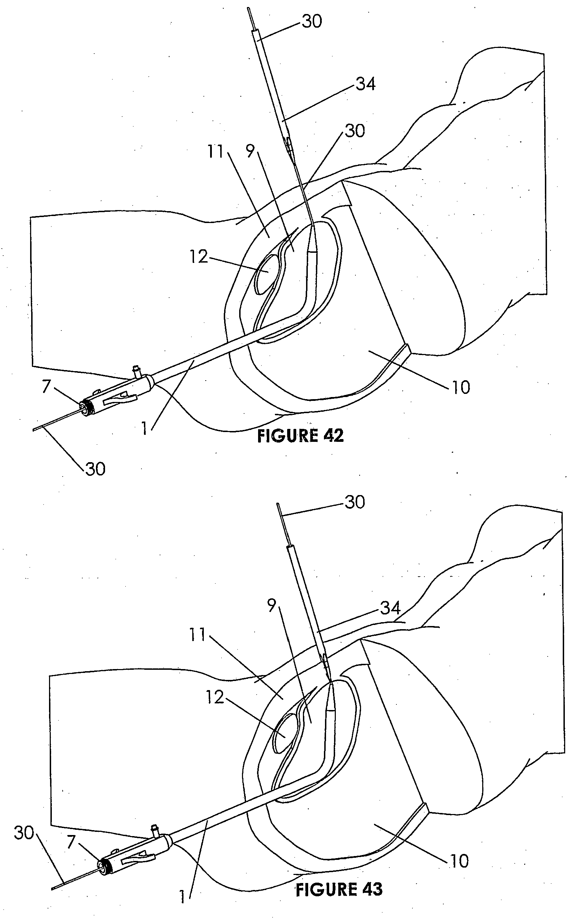

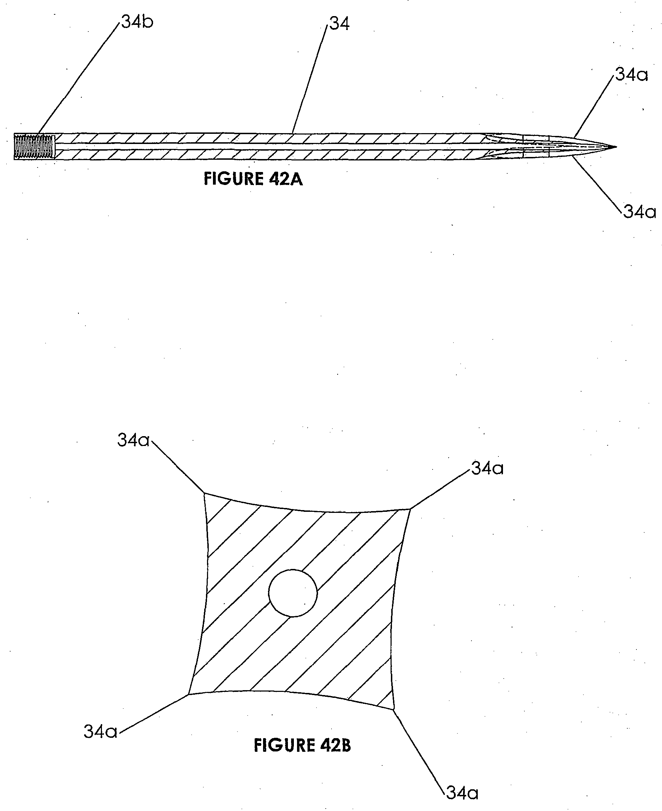

As shown in Figures 42 and 43, a trocar 34 may be used in lieu of a knife for a quicker and easier incision. The trocar 34 is loaded onto the wire and then pushed down until it makes contact with the tip 3 of the sound. Figure 42A is a section view of the trocar. As shown in this figure, the trocar comprises trocar blades 34a on one end and inner threads 34b on the other end. The inner threads allow the trocar to be attached to an extension if greater length is needed. Figure 42B is a perspective view of the trocar showing the scalloped blades. Figure 42C is a section view of the trocar, showing the cut made by the trocar blades. The trocar provides a different type of cut than the knife due to the way it is shaped. The present invention is not limited to any particular shape of the trocar or trocar blades.

Figure 44 is a section view of the screwdriver on a wire. The screwdriver may be used whenever the tip of the sound will not reach the skin. The screwdriver enables the physician to remove the tip of the sound so that a catheter can then be inserted over the wire into the vacated distal end of the sound. As shown in this figure, the screwdriver 35 is placed onto the wire 30 after the wire is passed out through the patient's abdomen and after an incision 36 has been made with one of the cutting devices depicted in Figures 40-43. The screwdriver 35 is then guided by the wire 30 as it is pushed through the abdominal wall 11. When the screwdriver 35 comes into contact with the tip 3 of the sound, as shown in Figure 45, the screwdriver is used to unscrew the removable tip 3 from the sound 1. In this procedure, a ball (not shown) or other nodule is placed on the wire and advanced to the tip of the sound so that the tip 3 can be removed from the patient's body by pulling the wire up through the incision site 36 after the tip 3 has been unscrewed, as shown in Figure 46. Once the tip 3 is removed, the catheter (not shown) can be placed on the wire (as described in connection with the ball-on-a-wire, hook-on-a-wire and nodule-on-a-wire embodiments described above) and the catheter tip advanced into the distal end of the sound (the end from which the tip was removed). At that point, the balloon is inflated to assist with lodging the catheter inside the sound. The catheter and sound are pulled back out through the urethra, the balloon is deflated, the wire with the nodule is pulled out the abdominal end of the sound, the catheter is separated from the sound, and the catheter is pulled back into the bladder. The balloon is then re-inflated to maintain the catheter in place, and the catheter is attached to drainage.

Figure 47 is a section view of a first exemplary embodiment of a sound that has been adapted to facilitate the balloon capture method. In this embodiment, as shown in Figures 2 and 3, the distal end of the sound 6 includes internal threads 6a into which a removable tip 3 (not shown) can be inserted; however, the improved sound also includes a plurality of internal ribs 39. The purpose of the ribs 39 is to hold the balloon 15 (not shown) in place after it is inflated. The circle around the distal end of the sound 6 in Figure 47 shows the orientation of Figure 48. Figure 48 is a partial section view of the sound shown in Figure 47. This figure shows the ribs 39 in greater detail.

Figure 49 is a section view of a second exemplary embodiment of a sound that has been adapted to facilitate the balloon capture method. In this embodiment, the distal end of the sound 6 includes internal threads 6a, as in the previous embodiment, but instead of the ribs of Figures 47 and 48, the sound has an internal flange 40 that prevents the balloon 15 (not shown) from being dislodged after it is inflated. In practice, the balloon 15 (not shown) would be inserted in a deflated state into the distal end of the sound 6 past the flange 40 and then inflated. The circle around the distal end of the sound 6 in Figure 49 shows the orientation of Figure 50. Figure 50 is a partial section view of the sound shown in Figure 49. This figure shows the internal flange 40 in greater detail.

Figures 51-74 depict yet another catheter capture device that consists of a lipped sound, obturator and retractable trocar. Figure 51 is a perspective view of the lipped sound 41 with the obturator 42, both of which are hollow. Only the tapered end 46 of the obturator 42 protrudes from the end of the sound 41. The circle at the distal end of the sound 41 in Figure 51 shows the orientation of Figure 52. Figure 52 is a partial perspective view of the tip of the lipped sound 41 and obturator 42 shown in Figure 51. This figure shows the triangular indentations 44 in the sound 41 and the hole 43 in the end of the obturator 42. The hole 43 in the end of the obturator extends the entire length of the obturator and allows for passage of a wire to be used in capturing the catheter, as described in connection with the ball-on-a-wire, hook-on-a-wire, and nodule-on-a-wire exemplary methods discussed above. The purpose of the triangular indentations 44 is discussed in connection with Figure 61.

Figure 53 is a section view of the obturator of Figure 51 fully inserted inside the sound. As shown in this figure, the obturator 42 comprises a plug 45, which prevents the obturator 42 from being pushed too far out the other end of the sound 41. The length of the obturator 42 is such that when the plug 45 comes into contact with the proximal end of the sound 7, only the tapered end 46 of the obturator 42 protrudes from the distal end of the sound 6.

Figure 54 is a section view of the obturator of Figure 51 partially inserted inside the sound. This figure is the same as Figure 53 except that the obturator 42 has been partially withdrawn. Figure 55 is a section view of the obturator of Figure 51 partially inserted inside the sound 41 but more fully withdrawn than in Figure 54. Figure 56 is a section view of the obturator of Figure 51 fully withdrawn from the sound 41.

Figure 57 is a rear perspective view of the obturator of Figure 51. This figure shows the plug 45, the tapered end 46 of the obturator, and one of the water channels 47 that runs longitudinally from one end of the obturator 42 to the other. The purpose of the water channels 47 is to allow water to be pumped into the bladder through the sound with the obturator in place inside the sound. Figure 57 also shows the wings 48, which fill in or insert into the triangular indentations 44 of the sound 41 when the obturator 42 is fully inserted. The purpose of the wings 48 is to prevent the triangular indentations from catching on anything as the sound and obturator are inserted into the patient's body.

Figure 58 is a front perspective view of the obturator of Figure 51. This figure shows the obturator with three water channels 47, but the present invention is not limited to any particular number of water channels. Preferably, the obturator has three or four water channels 47. Figure 59 is a top view of the obturator of Figure 51. It shows the water channels 47, tapered end 46 and plug 45.

Figure 60 is a perspective view of a lipped sound with the retractable trocar 49. The circle at the distal end of the sound 41 in Figure 60 shows the orientation of Figure 61. Figure 61 is a partial perspective view of the tip of the lipped sound 41 and retractable trocar 49 shown in Figure 60. Both the sound 41 and the retractable trocar 49 are hollow. This figure shows the cutting end 50 of the trocar 49, which comprises a plurality of blades 51. When the trocar 49 is fully inserted into the sound 41, the blades 51 fit into the triangular indentations 44. Although the indentations are shown as being triangular, they could be of any shape that accommodates the blades.

Figure 62 is a section view of the retractable trocar of Figure 60 fully inserted inside the sound. As shown in this figure, the retractable trocar 49 comprises a plug 45, which has the same purpose as discussed in connection with Figure 53.

Figure 63 is a section view of the retractable trocar of Figure 60 partially inserted inside the sound. This figure is the same as Figure 62 except that the trocar 49 has been partially withdrawn. Figure 64 is a section view of the retractable trocar of Figure 60 partially inserted inside the sound but more fully withdrawn than in Figure 63. Figure 65 is a section view of the retractable trocar of Figure 60 fully withdrawn from the sound.

Figure 66 is a rear perspective view of the retractable trocar of Figure 60. This figure shows the plug 45, the blades 51, and one of the water channels 47 that runs longitudinally from one end of the trocar 49 to the other. The purpose of the water channels 47 is the same as mentioned in connection with Figure 57.

Figure 67 is a front perspective view of the retractable trocar of Figure 60. This figure shows the water channels 47 more clearly than in Figure 66. The circle around the tip of the trocar in Figure 67 shows the orientation of Figure 68. Figure 68 is a partial perspective view of the tip of the retractable trocar of Figure 60. It shows the cutting end 50 of the trocar 49, including the blades 51, in greater detail. A guide wire may be inserted through the trocar to guide passage and would exit through the cutting end 50. The trocar is shown in Figures 67 and 68 with three water channels 47, but the present invention is not limited to any particular number of water channels 47. Preferably, the trocar has three or four water channels 47. Figure 69 is a top view and Figure 70 is a side view of the retractable trocar of Figure 60.

Figure 71 is a perspective view of the lipped sound of Figures 51-56 and 60-65. The circle at the distal end of the sound 41 in Figure 71 shows the orientation of Figure 72. Figure 72 is a partial perspective view of the tip of the lipped sound of Figure 71. Figure 72 shows more clearly the lip 52 and triangular indentations 44. The purpose of the lip is to hold the balloon 15 (not shown) in the catheter tip 14 (not shown) in place after the trocar is either partially or fully withdrawn and a catheter inserted into the sound. In practice, either the obturator 42 or retractable trocar 49, or both of them, could be used to punch (in the case of the obturator 42) or cut (in the case of the retractable trocar 49) a hole in the patient's abdomen from the inside-out. The obturator or trocar would then be either partially or completely withdrawn, and the catheter would be inserted inside the tip of the sound 41. The balloon 15 (not shown) would be inflated, and the lip 52 would prevent the catheter from slipping out of the sound after the balloon has been inflated.

Figure 73 is a section view of the lipped sound of Figures 51-56 and 60-65. The circle at the distal end of the sound 41 in Figure 73 shows the orientation of Figure 74. Figure 74 is a partial section view of the tip of the lipped sound of Figure 73. As in Figure 72, this figure shows the lip 52 and triangular indentations 44 into which the wings 48 of the obturator 42 and the blades 51 of the retractable trocar 49 fit.

Both the obturator 42 and trocar 49 are hollow so that they can be used in connection with the ball-on-a-wire, hook-on-a-wire and nodule-on-a-wire exemplary methods of capturing the catheter described above. In other words, the wire could be inserted through the obturator or trocar, as the case maybe, and out through the abdominal wall, where it could be used to capture the catheter, as described above.

Figures 75-93 relate to an alternative exemplary embodiment of a catheter capture device that consists of a sound, obturator and retractable trocar. In this embodiment, the sound does not have a lip (as in Figures 51-74), but the sound, obturator and retractable trocar are all hollow and can be used to capture the catheter with the ball-on-a-wire, hook-on-a-wire, and nodule-on-a-wire exemplary methods described above. This embodiment could also be used with the exemplary balloon inflation method, but there would be no lip to hold the balloon in place; instead, the balloon would be held in place by friction.

Figure 75 is a perspective view of a sound with an alternate embodiment of the obturator 54. This figure is the same as Figure 51, except that the sound 53 does not have a lip, and the obturator 54 does not have wings. As in Figure 51, both the sound and the obturator are hollow, and the obturator 43 comprises a hole 43 that extends the length of the obturator. When the obturator is fully inserted inside the sound, as shown in Figure 51, only the tapered end 46 of the obturator 54 protrudes from the end of the sound 41. The circle at the distal end of the sound 53 in Figure 75 shows the orientation of Figure 76. Figure 76 is a partial perspective view of the tip of the sound and obturator shown in Figure 75. This figure is similar to Figure 52 except that the sound 53 has no lip, and the obturator 54 does not have wings.

Figure 77 is a section view of the obturator of Figure 75 fully inserted inside the sound. Figure 78 is a section view of the obturator of Figure 75 partially inserted inside the sound. Figure 79 is a section view of the obturator of Figure 75 partially inserted inside the sound but more fully withdrawn than in Figure 78. Figure 80 is a section view of the obturator of Figure 75 fully withdrawn from the sound.

Figure 81 is a rear perspective view of the obturator of Figure 75. Figure 82 is a front perspective view of the obturator of Figure 75. Figure 83 is a top view of the obturator of Figure 75. These figures are similar to Figures 57, 58 and 59, respectively, except that the obturator 54 has no wings.

Figure 84 is a perspective view of a sound with an alternate exemplary embodiment of the retractable trocar 53. The trocar shown in Figure 84 has an angled cutting edge 56. Although the cutting edge 56 is shown as angled, it is not limited to any particular angle or shape of cutting edge, as long as it has the ability to cut through skin and bodily tissue. The circle at the distal end of the sound 53 in Figure 84 shows the orientation of Figure 85. Figure 85 is a partial perspective view of the tip of the sound and retractable trocar shown in Figure 84. Figures 84 and 85 are similar to Figures 60 and 61, respectively, except that the sound 53 has no lip, and the trocar 55 has an angled cutting edge 56 instead of the plurality of blades shown in Figure 61. Figure 85 also shows the hole 43 in the end of the trocar. The hole 43 extends the entire length of the trocar, in order to accommodate a wire for purposes of catheter capture.

Figure 86 is a section view of the retractable trocar of Figure 84 fully inserted inside the sound. Figure 87 is a section view of the retractable trocar of Figure 84 partially inserted inside the sound. Figure 88 is a section view of the retractable trocar of Figure 84 partially inserted inside the sound but more fully withdrawn than in Figure 87. Figure 89 is a section view of the retractable trocar of Figure 84 fully withdrawn from the sound.

Figure 90 is a rear perspective view of the retractable trocar of Figure 84. Figure 91 is a front perspective view of the retractable trocar of Figure 84. Figure 92 is a top view and Figure 93 is a side view of the retractable trocar of Figure 84. These figures are similar to Figures 66, 67, 69 and 70, respectively, except that the retractable trocar 55 has an angled cutting edge 56 instead of the plurality of blades shown in Figure 68.

Like the obturator and retractable trocar shown in Figures 58 and 67, respectively, the obturator and retractable trocar shown in Figures 82 and 91 are hollow (to accommodate a wire for capturing the catheter or for assisting in guiding and advancing the sound through to the skin level), and they also have water channels 47. These two features are common to both exemplary embodiments of the obturator and retractable trocar described herein.

Although several preferred embodiments of the present invention have been shown and described, it will be apparent to those skilled in the art that many changes and modifications may be made without departing from the invention in its broader aspects. The appended claims are therefore intended to cover all such changes and modifications as fall within the scope of the invention.

### REFERENCES

(1) Raz, S., Campbell's Urology, 7th Ed., Vol. 1, p. 1092 (WB Saunders, 1998).
(2) Hendrix, S. L. et al., "Pelvic Organ Prolapse in the Women's Health Initiative: Gravity and Gravidity," Am. J. Obstet. Gynecol. 186(6): 1160-66.
(3) Waetjen et al., "Stress Urinary Incontinence Surgery in the United States," Obstet. Gynecol. 101(4): 671-75 (2003).
(4) Olsen et al., "Epidemiology of Surgically Managed Pelvic Organ Prolapse and Urinary Incontinence," Obstet. Gynecol. 89: 501-06 (1997).
(5) Wahle, G. et al., "Complications of Vaginal Surgery," in Raz, S., Female Urology, 2nd Ed., pp. 623-24 (1996).
(6) Kobashi, K. and Leach, G., "Pelvic Prolapse," J. Urol 164(6): 1984 (2000).

### DEFINITIONS

The term "cannula" means a flexible tube, usually containing a trocar at one end, that is inserted into a bodily cavity, duct, or vessel to drain fluid or administer a substance such as a medication.

The term "catheter" means a hollow flexible tube for insertion into a body cavity, duct or vessel to allow the passage of fluids or distend a passageway.

The term "cystotomy" means a procedure in which an incision is made into the bladder.

The term "ISC" means intermittent self-catheterization.

The term "obturator" means a structure that removably closes or obstructs.

The term "PUR" means post-operative urinary retention.

The term "sound" means any elongated instrument or probe, usually metallic, by which cavities of the body are sounded or explored.

The term "SUI" means stress urinary incontinence.

The term "trocar" means a sharply pointed instrument, usually with an attached cannula, used to perforate a hollow organ and drain fluid.

## Claims

1. Apparatus for creating a suprapubic incision (36) through an abdominal wall (11) into a bladder (9) and for placing a catheter (13) in the incision (36), comprising in combination:
a hollow urethral sound (1) having a distal end (6) adapted for insertion within a urethra and the bladder (9) and a proximal end (2) adapted to remain outside of the urethra by which to manipulate the distal end (6) of the sound (1) within the bladder (9), the distal end (6) of the sound (1) having a sound tip (3);
a wire (30) having proximal and distal ends and positioned within the sound (1) to move between an extended position and a retracted position, the extended position extending the distal end of the wire (30) from the sound tip (3) sufficiently to penetrate from the bladder (9) through the abdominal wall (11) and to expose a portion of the distal end of the wire (30) outside of the abdominal wall (11), the retracted position retracting the distal end of the wire (30) from the extended position toward the sound tip (3), the distal end of the wire in the extended position establishing a path for the incision (36), the movement of the wire (30) from the extended position to the retracted position guiding a distal end (14, 15) of the catheter (13) from outside the abdominal wall (11) through the incision (36) and into the bladder (9);
a cutting tool (33, 34) adapted to create the incision (36) by movement along the path of the wire (30) through the abdominal wall (11) and into the bladder (9) to the sound tip (3), the cutting tool (33, 34) is also adapted to be moved out of the incision (36) along the path of the wire (30) after creating the incision;
the catheter (13) having a distal end (14, 15) and a proximal end (18) and a hollow portion extending between the distal end (14, 15) and the proximal end (18), the distal end (14, 15) of the catheter (13) having a hole (16) which allows liquid to flow into the distal end and through the hollow portion to the proximal end (18) of the catheter, the distal end of the catheter adapted to be placed through the incision (36) and into the bladder (9) while the proximal end (18) of the catheter remains outside of the incision (36) and the abdominal wall (11), the distal end of the catheter including a catheter tip (14) and an inflatable balloon (15); and
**characterized in that**:
the cutting tool (33, 34) is adapted to be guided from outside of the abdominal wall (11) along the path of the wire (30), and the cutting tool (33, 34) is also adapted to be removed from the path of the wire (30) from the outside of the abdominal wall (11) after creating the incision (36);
a removable connector (31, 32) adapted to selectively connect to the distal end of the wire (30) when the wire is in the extended position, the movement of the wire (30) from the extended position toward the retracted position contacting the connector (31, 32) with the catheter tip (14) and guiding the distal end (14, 15) of the catheter (13) from outside the abdominal wall (11) through the incision (36) and into the bladder (9), the movement of the distal end of the wire (30) from the retracted position bach to the extended position disconnecting the connector (31, 32) from the distal end of the catheter (13) within the bladder (9), the removal of the remaining wire in a distal direction from the incision disconnecting the catheter and the sound and allowing removal of the sound (1) from the urethra;
the removable connector (31, 32) permits the balloon (15) to be inflated within the bladder (9) while the catheter tip (14) remains connected to the connector (31, 32) on the distal end of the wire (30) within the bladder; and
the removable connector (31, 32) permits liquid from the bladder (9) to flow through the hole (16) in the catheter tip (14) into the hollow portion of the catheter (13) to the proximal end (18) while the catheter tip (14) remains connected to the connector (31, 32) on the distal end of the wire (30) within the bladder (9).

2. Apparatus as defined in claim 1, wherein:
the distal end of the wire (30) is extended and retracted by manipulation of the proximal end of the wire at the proximal end (2) of the sound (1).

3. Apparatus as defined in claim 1, wherein:
the cutting tool (33, 34) slides along the path of the wire (30) to create the incision (36).

4. Apparatus as defined in claim 3, wherein the cutting tool (33, 34) includes an opening through into which the distal end of the wire (30) is inserted to connect the cutting tool to the wire.

5. Apparatus as defined in claim 1, wherein the cutting tool includes a trocar (34).

6. Apparatus as defined in claim 1, wherein the cutting tool includes a knife (35).

7. Apparatus as defined in claim 1, wherein the removable connector includes a rotatable portion.

8. Apparatus as defined in claim 1, wherein the removable connector includes a threaded portion.

9. Apparatus as defined in claim 1, wherein the removable connector comprises an object (31, 32) on the wire (30) which is removably lodged in the catheter tip (3).

10. Apparatus as defined in claim 9, wherein the object is a ball (31).

11. Apparatus as defined in claim 9, wherein the object is a nodule.

12. Apparatus as defined in claim 9, wherein the object is a hook (32).

13. Apparatus as defined in claim 9, wherein the object is a crimp in the wire (30).

## Patentansprüche

1. Vorrichtung zum Erzeugen eines suprapubischen Einschnitts (36) durch eine Bauchdecke (11) in eine Blase (9) und zum Platzieren eines Katheters (13) in dem Einschnitt (36), die in Kombination Folgendes umfasst:
eine hohle Harnröhrensonde (1) mit einem zum Einführen in eine Harnröhre und die Blase (9) ausgelegten distalen Ende (6) und einem zum Verbleiben außerhalb der Harnröhre ausgelegten proximalen Ende (2), mit dem das distale Ende (6) der Sonde (1) in der Blase (9) manipuliert werden kann, wobei das distale Ende (6) der Sonde (1) eine Sondenspitze (3) aufweist;
einen Draht (30) mit einem proximalen und einem distalen Ende, in der Sonde (1) zum Bewegen zwischen einer ausgefahrenen Position und einer zurückgezogenen Position positioniert, wobei das distale Ende des Drahtes (30) in der ausgefahrenen Position von der Sondenspitze (3) ausreichend weit ausgefahren ist, um von der Blase (9) die Bauchdecke (11) zu penetrieren und einen Teil des distalen Endes des Drahtes (30) außerhalb der Bauchdecke (11) zu exponieren, wobei in der zurückgezogenen Position das distale Ende des Drahtes (30) von der ausgefahrenen Position in Richtung der Sondenspitze (3) zurückgezogen ist, wobei das distale Ende des Drahtes in der ausgefahrenen Position einen Pfad für den Einschnitt (36) erzeugt, wobei die Bewegung des Drahtes (30) von der ausgefahrenen Position in die zurückgezogene Position ein distales Ende (14; 15) des Katheters (13) von außerhalb der Bauchdecke (11) durch den Einschnitt (36) in die Blase (9) führt;
ein Schneidinstrument (33, 34) zum Erzeugen des Einschnitts (36) durch eine Bewegung entlang dem Pfad des Drahtes (30) durch die Bauchdecke (11) in die Blase (9) zur Sondenspitze (3), wobei das Schneidinstrument (33, 34) auch zum Bewegen aus dem Einschnitt (36) entlang dem Pfad des Drahtes (30) nach dem Erzeugen des Einschnitts ausgelegt ist;
wobei der Katheter (13) ein distales Ende (14, 15) und ein proximales Ende (18) und einen zwischen dem distalen Ende (14, 15) und dem proximalen Ende (18) verlaufenden hohlen Abschnitt aufweist, wobei das distale Ende (14, 15) des Katheters (13) ein Loch aufweist, das einen Fluss von Flüssigkeit in das distale Ende und durch den hohlen Abschnitt zum proximalen Ende (18) des Katheters zulässt, wobei das distale Ende des Katheters zum Platzieren durch den Einschnitt (38) in der Blase (9) ausgelegt ist, während das proximale Ende (18) des Katheters außerhalb des Einschnitts (36) und der Bauchdecke (11) bleibt, wobei das distale Ende des Katheters eine Katheterspitze (14) und einen aufblasbaren Ballon (15) aufweist; und
**dadurch gekennzeichnet, dass**:
das Schneidinstrument (33, 34) zum Führen von außerhalb der Bauchdecke (11) entlang dem Pfad des Drahtes (30) ausgelegt ist und das Schneidinstrument (33, 34) auch zum Entfernen aus dem Pfad des Drahtes (30) von außerhalb der Bauchdecke (11) nach dem Erzeugen des Einschnitts (36) ausgelegt ist;
einen entfernbaren Verbinder (31, 32), ausgelegt zum selektiven Verbinden mit dem distalen Ende des Drahtes (30), wenn der Draht in der ausgefahrenen Position ist, wobei die Bewegung des Drahtes (30) von der ausgefahrenen Position in die zurückgezogene Position den Verbinder (31, 32) mit der Katheterspitze (14) berührt und das distale Ende (14, 15) des Katheters (13) von außerhalb der Bauchdecke (11) durch den Einschnitt (36) in die Blase (9) führt, wobei die Bewegung des distalen Endes des Drahtes (30) von der zurückgezogenen Position zurück in die ausgefahrene Position den Verbinder (31, 32) vom distalen Ende des Katheters (13) in der Blase (9) trennt, wobei das Entfernen des restlichen Drahtes in einer distalen Richtung vom Einschnitt den Katheter und die Sonde trennt und ein Entfernen der Sonde (1) aus der Harnröhre zulässt;
der entfernbare Verbinder (31, 32) das Aufblasen des Ballons (15) in der Blase (9) zulässt, während die Katheterspitze (14) mit dem Verbinder (31, 32) am distalen Ende des Drahtes (30) in der Blase verbunden bleibt; und
der entfernbare Verbinder (31, 32) einen Fluss von Flüssigkeit von der Blase (9) durch das Loch (16) in der Katheterspitze (14) in den hohlen Abschnitt des Katheters (13) zum proximalen Ende (18) zulässt, während die Katheterspitze (14) mit dem Verbinder (31, 32) am distalen Ende des Drahtes (30) in der Blase (9) verbunden bleibt.

2. Vorrichtung nach Anspruch 1, wobei:
das distale Ende des Drahtes (30) durch Manipulieren des proximalen Endes des Drahtes am proximalen Ende (2) der Sonde (1) ausgefahren und zurückgezogen wird.

3. Vorrichtung nach Anspruch 1, wobei:
das Schneidinstrument (33, 34) entlang dem Pfad des Drahtes (30) gleitet, um den Einschnitt (36) zu erzeugen.

4. Vorrichtung nach Anspruch 3, wobei das Schneidinstrument (33, 34) eine Öffnung aufweist, durch die das distale Ende des Drahtes (30) eingeführt wird, um das Schneidinstrument mit dem Draht zu verbinden.

5. Vorrichtung nach Anspruch 1, wobei das Schneidinstrument einen Trokar (34) beinhaltet.

6. Vorrichtung nach Anspruch 1, wobei das Schneidinstrument ein Messer (35) beinhaltet.

7. Vorrichtung nach Anspruch 1, wobei der entfernbare Verbinder einen drehbaren Abschnitt aufweist.

8. Vorrichtung nach Anspruch 1, wobei der entfernbare Verbinder einen Gewindeabschnitt aufweist.

9. Vorrichtung nach Anspruch 1, wobei der entfernbare Verbinder ein entfernbar in der Katheterspitze (3) untergebrachtes Objekt (31, 32) an dem Draht (30) umfasst.

10. Vorrichtung nach Anspruch 9, wobei das Objekt eine Kugel (31) ist.

11. Vorrichtung nach Anspruch 9, wobei das Objekt ein Knötchen ist.

12. Vorrichtung nach Anspruch 9, wobei das Objekt ein Haken (32) ist.

13. Vorrichtung nach Anspruch 9, wobei das Objekt ein Knick im Draht (30) ist.

## Revendications

1. Appareil destiné à créer une incision suprapubique (36) à travers une paroi abdominale (11) jusque dans une vessie (9), et à placer un cathéter (13) dans l'incision (36), comprenant en combinaison :
une sonde urétrale creuse (1) possédant une extrémité distale (6) conçue en vue d'une insertion à l'intérieur d'une urètre et de la vessie (9), et une extrémité proximale (2), conçue pour rester à l'extérieur de l'urètre, laquelle permet de manipuler l'extrémité distale (6) de la sonde (1) à l'intérieur de la vessie (9), l'extrémité distale (6) de la sonde (1) possédant une pointe de sonde (3) ;
un fil métallique (30) possédant des extrémités proximale et distale et positionné à l'intérieur de la sonde (1) afin de se déplacer entre une position déployée et une position rétractée, la position déployée allongeant l'extrémité distale du fil métallique (30) depuis la pointe de la sonde (3) de manière suffisante pour pénétrer à partir de la vessie (9) à travers la paroi abdominale (11) et pour exposer une portion de l'extrémité distale du fil métallique (30) à l'extérieur de la paroi abdominale (11), la position rétractée ramenant l'extrémité distale du fil métallique (30) depuis la position déployée vers la pointe de la sonde (3), l'extrémité distale du fil métallique dans la position déployée établissant un trajet pour l'incision (36), alors que le mouvement du fil métallique (30) à partir de la position déployée vers la position rétractée guide une extrémité distale (14, 15) du cathéter (13) depuis la face extérieure de la paroi abdominale (11) à travers l'incision (36) et jusque dans la vessie (9) ;
un outil de coupe (33, 34) conçu pour créer l'incision (36) grâce au mouvement le long du trajet du fil métallique (30) à travers la paroi abdominale (11) et jusque dans la vessie (9) vers la pointe de la sonde (3), l'outil de coupe (33, 34) étant également conçu pour être déplacé pour sortir de l'incision (36) le long du trajet du fil métallique (30) après avoir créé l'incision ;
le cathéter (13) possédant une extrémité distale (14, 15) et une extrémité proximale (18) et une portion creuse laquelle s'étend entre l'extrémité distale (14, 15) et l'extrémité proximale (18), l'extrémité distale (14, 15) du cathéter (13) possédant un trou (16) qui permet à du liquide de couler jusque dans l'extrémité distale et à travers la portion creuse vers l'extrémité proximale (18) du cathéter, l'extrémité distale du cathéter étant conçue pour être placée à travers l'incision (36) et jusque dans la vessie (9) tandis que l'extrémité proximale (18) du cathéter rester à l'extérieur de l'incision (36) et de la paroi abdominale (11), l'extrémité distale du cathéter incluant une pointe de cathéter (14) et un ballon gonflable (15) ; et
**caractérisé en ce que** :
l'outil de coupe (33, 34) est conçu pour être guidé à partir de la face extérieure de la paroi abdominale (11) le long du trajet du fil métallique (30), et l'outil de coupe (33, 34) est également conçu pour être enlevé du trajet du fil métallique (30) à partir de la face extérieure de la paroi abdominale (11) après la création de l'incision (36) ;
un raccord amovible (31, 32) est conçu pour être raccordé sélectivement à l'extrémité distale du fil métallique (30) lorsque le fil métallique se trouve dans la position déployée, le mouvement du fil métallique (30) depuis la position déployée vers la position rétractée mettant en contact le raccord (31, 32) et la pointe du cathéter (14) et guidant l'extrémité distale (14, 15) du cathéter (13) depuis la face extérieure de la paroi abdominale (11) à travers l'incision (36) et jusque dans la vessie (9), le mouvement de l'extrémité distale du fil métallique (30) à partir de la position rétractée en retour vers la position déployée provoquant le débranchement du raccord (31, 32) au niveau de l'extrémité distale du cathéter (13) à l'intérieur de la vessie (9), l'enlèvement du fil métallique restant suivant un sens distal au niveau de l'incision provoquant le débranchement du cathéter et de la sonde et permettant l'enlèvement de la sonde (1) au niveau de l'urètre ;
le raccord amovible (31, 32) permet le gonflage du ballon (15) à l'intérieur de la vessie (9), pendant que la pointe du cathéter (14) reste branchée au raccord (31, 32) sur l'extrémité distale du fil métallique (30) à l'intérieur de la vessie ; et
le raccord amovible (31, 32) permet au liquide provenant de la vessie (9) de s'écouler à travers le trou (16) pratiqué dans la pointe du cathéter (14) jusque dans la portion creuse du cathéter (13) vers l'extrémité proximale (18), pendant que la pointe du cathéter (14) reste raccordée au raccord (31, 32) sur l'extrémité distale du fil métallique (30) à l'intérieur de la vessie (9).

2. Appareil selon la revendication 1 :
l'extrémité distale du fil métallique (30) étant déployée et rétractée en vertu d'une manipulation de l'extrémité proximale du fil métallique au niveau de l'extrémité proximale (2) de la sonde (1).

3. Appareil selon la revendication 1 :
l'outil de coupe (33, 34) coulissant le long du trajet du fil métallique (30) afin de créer l'incision (36).

4. Appareil selon la revendication 3, l'outil de coupe (33, 34) incluant une ouverture à travers laquelle l'extrémité distale du fil métallique (30) est insérée afin de raccorder l'outil de coupe au fil métallique.

5. Appareil selon la revendication 1, l'outil de coupe incluant un trocart (34).

6. Appareil selon la revendication 1, l'outil de coupe incluant une lame tranchante (35).

7. Appareil selon la revendication 1, le raccord amovible incluant une portion apte à tourner.

8. Appareil selon la revendication 1, le raccord amovible incluant une portion filetée.

9. Appareil selon la revendication 1, le raccord amovible comprenant un objet (31, 32) sur le fil métallique (30) qui est logé de manière amovible dans la pointe du cathéter (3).

10. Appareil selon la revendication 9, l'objet étant une bille (31).

11. Appareil selon la revendication 9, l'objet étant un nodule.

12. Appareil selon la revendication 9, l'objet étant un crochet (32).

13. Appareil selon la revendication 9, l'objet étant une sertissure dans le fil métallique (30).
